(19) Europäisches Patentamt
European Patent Office
Office européen des brevets

(11) Veröffentlichungsnummer : **0 103 537**
**B1**

(12) **EUROPÄISCHE PATENTSCHRIFT**

(45) Veröffentlichungstag der Patentschrift : **08.07.87**

(51) Int. Cl.⁴ : **C 07 D 249/14**, A 01 N 47/36

(21) Anmeldenummer : **83810315.8**

(22) Anmeldetag : **11.07.83**

(54) **N-Arylsulfonyl-N'-triazolylharnstoffe.**

(30) Priorität : **16.07.82 CH 4358/82**
**02.12.82 CH 7009/82**
**10.06.83 CH 3186/83**

(43) Veröffentlichungstag der Anmeldung : **21.03.84 Patentblatt 84/12**

(45) Bekanntmachung des Hinweises auf die Patenterteilung : **08.07.87 Patentblatt 87/28**

(84) Benannte Vertragsstaaten :
**AT BE CH DE FR GB IT LI NL SE**

(56) Entgegenhaltungen :
**EP-A- 0 029 663**
**EP-A- 0 030 142**
**EP-A- 0 073 562**

(73) Patentinhaber : **CIBA-GEIGY AG**
**Klybeckstrasse 141**
**CH-4002 Basel (CH)**

(72) Erfinder : **Töpfl, Werner, Dr.**
**Dorneckstrasse 68**
**CH-4143 Dornach (CH)**
Erfinder : **Kristinsson, Haukur, Dr.**
**Kreuzackerweg 19**
**CH-4103 Bottmingen (CH)**
Erfinder : **Meyer, Willy**
**Talweg 49**
**CH-4125 Riehen (CH)**

**EP 0 103 537 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung umfasst eine Gruppe neuer untenstehend definierter N-Arylsulfonyl-N'-triazol-harnstoffe, die wertvolle pflanzenwachstumregulierende und pflanzenbakterizide Eigenschaften aufweisen und sich zur gezielten Beeinflussung des Pflanzenwuchses, zur Bekämpfung von Unkräutern z. B. in Kulturen von Nutzpflanzen und/oder zur Bekämpfung phytopathogener Bakterien eignen. Sie umfasst ferner die Herstellung dieser Sulfonylharnstoffe, sowie landwirtschaftlich nutzbare Mittel, die diese Substanzen als Wirkstoff enthalten.

Die neuen erfindungsgemässen N-Arylsulfonyl-N'-triazolyl-harnstoffe entsprechen der allgemeinen Formel I

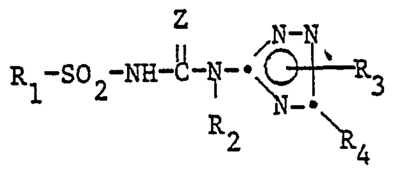

(I)

worin

$R_1$ einen Rest der Formel

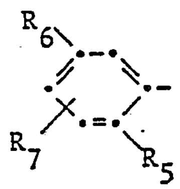

oder

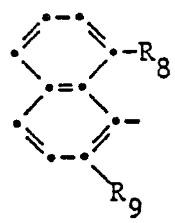

$R_2$ und $R_3$ unabhängig voneinander Wasserstoff oder $C_1$-$C_6$-Alkyl,

$R_4$ Wasserstoff, Halogen, $C_1$-$C_6$-Alkyl, $C_1$-$C_6$-Halogenalkyl, Cyclopropyl, —$NR_{10}R_{11}$ oder —X—$R_{12}$ und

Z Sauerstoff oder Schwefel bedeuten,

wobei

$R_5$ und $R_9$ Wasserstoff, Halogen, Cyan, Nitro, $C_1$-$C_6$-Alkyl, $C_1$-$C_6$-Halogenalkyl, gegebenenfalls durch 1 bis 3 Halogenatome substituiertes $C_2$-$C_6$-Alkenyl, —CO—$R_{13}$, —$NR_{10}R_{11}$, —$SO_2$—$C_1$-$C_4$-Halogenalkoxy, —$SO_2$—$NR_{10}R_{11}$, —NH—$SO_2$—$R_{15}$, —O—$SO_2$—$R_{15}$, —N($C_1$-$C_3$-Alkyl)—$SO_2$-$R_{15}$, oder einen gegebenenfalls durch einen oder mehrere Substituenten aus der Reihe Halogen, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Halogenalkoxy, $C_1$-$C_4$-Halogenalkylthio, $C_1$-$C_4$-Halogenalkylsulfinyl oder $C_1$-$C_4$-Halogensulfonyl substituierten Rest —Y—$R_{14}$,

$R_6$ Wasserstoff, Halogen, Nitro oder $C_1$-$C_6$-Alkoxy,

$R_7$ und $R_8$ Wasserstoff, Halogen, Nitro, $C_1$-$C_6$-Halogenalkyl, —$NH_2$, $C_1$-$C_6$-Alkyl, $C_1$-$C_6$-Alkoxy, $C_1$-$C_6$-Alkylthio, $C_1$-$C_6$-Alkylsulfinyl oder $C_1$-$C_6$-Alkylsulfonyl,

$R_{10}$ und $R_{11}$ unabhängig voneinander Wasserstoff, $C_1$-$C_4$-Alkyl oder Cyclopropyl oder $R_{10}$ und $R_{11}$ zusammen eine gegebenenfalls durch Methyl substituierte oder Sauerstoff, Schwefel, —SO—, —$SO_2$—, —NH— oder —N($C_1$-$C_4$-Alkyl)- unterbrochene $C_4$-$C_6$-Alkylenbrücke,

$R_{12}$ $C_1$-$C_4$-Alkyl, $C_1$-$C_4$-Halogenalkyl oder Alkoxyalkyl mit höchstens 4 Kohlenstoffatomen,

$R_{13}$ Wasserstoff, $C_1$-$C_4$-Alkyl, $C_3$-$C_6$-Alkenyloxy, $C_3$-$C_6$-Alkinyloxy, $C_1$-$C_4$-Halogenalkyl, $C_1$-$C_6$-Alkylthio, Phenoxy, Benzyloxy, —$NR_{10}R_{11}$ oder gegebenenfalls durch 1 bis 3 Halogenatome substituiertes $C_1$-$C_6$-Alkoxy,

$R_{14}$ $C_1$-$C_6$-Alkyl, $C_2$-$C_6$-Alkenyl oder $C_3$-$C_6$-Alkinyl

$R_{15}$ $C_1$-$C_6$-Alkyl oder $C_1$-$C_6$-Halogenalkyl und

X und Y unabhängig voneinander Sauerstoff, Schwefel, eine Sulfinyl- oder Sulfonylbrücke bedeuten, sowie Salze dieser Verbindungen ; mit der Massgabe, dass

a) $R_1$ nur dann die angegebene Bedeutung hat, wenn $R_4$ für die Gruppe —X—$CHF_2$ steht, und dass

b) $R_4$ nur dann die angegebene Bedeutung hat, wenn $R_1$ für den substituierten Phenylkern steht und $R_5$ für die Gruppe —Y—$C_2$-$C_6$-Halogenalkenyl stehen.

Phenylsulfonylharnstoffe mit pflanzenwuchsregulierender und pharmazeutischer Wirkung sind aus den publizierten Europäischen Patentanmeldungen EP-A-29 663 und EP-A-30 142 bekannt. Der von der Formel I ausgeklammerte Teil ist Bestandteil der Zwischenpublikation EP-A-73 562.

Die vorliegende Erfindung umfasst somit neben den freien Wirkstoffen auch die auf dem Agrargebiet verwendbaren Salze der Verbindungen der Formel I.

Unter Alkyl oder Alkylanteil eines anderen Substituenten sind je nach Zahl der angegebenen Kohlenstoffatome beispielsweise folgende Gruppen zu verstehen : Methyl, Aethyl, Propyl, Butyl, Pentyl oder Hexyl, sowie ihre Isomeren wie Isopropyl, Isobutyl, tert.-Butyl, Isopentyl usw. Halogenalkyl steht für einen ein- oder mehrfach durch Halogen substituierten Alkylrest, beispielsweise für $CF_3$, $CH_2Cl$, $CCl_3$, $CHCl_2$, $C_2H_4Cl$, $C_2H_4Br$, $CHF_2$, $CH_2F$, $—CF_2—CF_2H$, $—C_2F_5$ usw. Alkenyl steht z. B. für Vinyl, Propenyl-(1), Allyl, Butenyl-(1), Butenyl-(2), Butenyl-(3) etc. Alkinyl bedeutet insbesondere Propargyl. Halogen steht hier und im folgenden für Fluor, Chlor, Brom oder Jod, vorzugsweise für Chlor oder Fluor. Als $C_4$-$C_6$-Alkylenbrücke wird die Tetra-, Penta- oder Hexamethylengruppe bezeichnet. Unter den in die Definitionen für $R_4$ fallenden Halogenalkoxyresten versteht man beispielsweise Chlormethoxy, Fluormethoxy, Difluormethoxy, Trifluormethoxy, 2-Chloräthoxy, 2-Fluoräthoxy, 2,2,2-Trifluoräthoxy, 1,1,2,2-Tetrafluoräthoxy oder Perfluoräthoxy. Bevorzugt ist Difluormethoxy. Beispiele für unter die Definition von $R_5$ fallenden Halogenalkenyloxyreste sind 1-Chlorvinyloxy, 2-Chlorvinyloxy, 1,2-Dichlorvinyloxy, 2,2-Dichlorvinyloxy, 1,2,2-Trichlorvinyloxy, 2-Chlorallyloxy, 2,3-Dichlorallyloxy, 2,3,3-Trichlorallyloxy oder 3,3,3-Trifluor-1-propenyloxy. Bevorzugt ist 1,2-Dichlorvinyloxy.

Die auf dem Agrargebiet einsetzbaren Salze sind z. B. diejenigen, die die Verbindungen der Formel I mit Aminen, Alkali- und Erdalkalimetallbasen oder quaternären Ammoniumbasen bilden. Hierbei sind unter Alkali- oder Erdalkalibasen besonders die Hydroxide von Lithium, Natrium, Kalium, Magnesium oder Calcium zu verstehen.

Beispiele für zur Salzbildung geeignete Amine sind primäre, sekundäre und tertiäre aliphatische und aromatische Amine wie Methylamin, Aethylamin, Propylamin, i-Propylamin, die vier isomeren Butylamine, Dimethylamin, Diäthylamin, Diäthanolamin, Dipropylamin, Diisopropylamin, Di-n-butylamin, Pyrrolidin, Piperidin, Morpholin, Trimethylamin, Triäthylamin, Tripropylamin, Chinuclidin, Pyridin, Chinolin und i-Chinolin, insbesondere aber Aethyl-, Propyl-, Diäthyl- oder Triäthylamin, vor allem aber iso-Propylamin und Diäthanolamin.

Beispiele für quaternäre Ammoniumbasen sind im allgemeinen die Kationen von Halogenammoniumsalzen, z. B. das Tetramethylammoniumkation, das Trimethylbenzylammoniumkation, das Triäthylbenzylammoniumkation, das Tetraäthylammoniumkation, das Trimethyläthylammoniumkation, aber auch das Ammoniumkation.

Die Verbindungen der Formel I sind bei Raumtemperatur stabile/biologisch aktive Harze oder Feststoffe, die sich durch sehr wertvolle pflanzenwachstumsregulierende, vor allem wachstumshemmende und pflanzenbakterizide, vor allem gegen phytopathogene Bakterien aus den Familien Xanthomonadaceae und Pseudomonadaceae, Eigenschaften auszeichnen. Sie lassen sich daher auf dem Agrarsektor oder auf verwandten Anwendungsgebieten sowohl zur gezielten Reduktion des Wachstums monokotyler und dikotyler Pflanzen einsetzen, wobei sie je nach Art der Verwendung bzw. der Aufwandmenge selektiv herbizide bis totalherbizide Eigenschaften enthalten, als auch zur Bekämpfung von phytopathogenen Bakterien aus den Familien Xanthomonodaceae und Pseudomonadaciae, wie beispielsweise Xanthomonas oryzae, Xanthomonas vesicatoria und Pseudomonas tomato.

Hervorzuheben ist auch die Gruppe von Verbindungen der Formel I worin

$R_1$ einen Rest der Formel

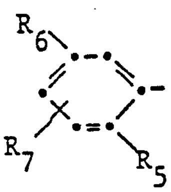

oder

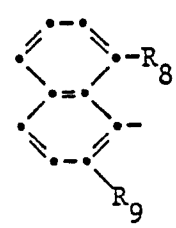

$R_2$ und $R_3$ unabhängig voneinander Wasserstoff oder $C_1$-$C_4$-Alkyl,

$R_4$ Wasserstoff, Halogen, $C_1$-$C_4$-Alkyl, $C_1$-$C_4$-Halogenalkyl, Cyclopropyl, $—NR_{10}R_{11}$ oder $—X—R_{12}$ und

Z Sauerstoff oder Schwefel bedeuten,

wobei

$R_5$ Wasserstoff, Halogen, Cyan, Nitro, $C_1$-$C_4$-Alkyl, $C_1$-$C_4$-Halogenalkyl, $C_2$-$C_6$-Alkenyl, $—CO—R_{13}$, $—NR_{10}R_{11}$ oder einen gegebenenfalls durch einen oder mehrere Substituenten aus der Reihe Halogen, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Halogenalkoxy, $C_1$-$C_4$-Halogenalkylthio, $C_1$-$C_4$-Halogenalkylsulfinyl oder $C_1$-$C_4$-Halogensulfonyl substituierten Rest $—Y—R_{14}$,

$R_6$ Wasserstoff, Halogen, Nitro oder $C_1$-$C_4$-Alkoxy,

$R_7$ Wasserstoff, Halogen, Nitro, $C_1$-$C_4$-Halogenalkyl, $—NH_2$, $C_1$-$C_4$-Alkyl, $C_1$-$C_4$-Alkoxy, $C_1$-$C_3$-Alkylthio, $C_1$-$C_3$-Alkylsulfinyl oder $C_1$-$C_3$-Alkylsulfonyl,

$R_{10}$ und $R_{11}$ unabhängig voneinander Wasserstoff, $C_1$-$C_3$-Alkyl, Cyclopropyl, $C_2$-$C_6$-Alkenyl oder

$C_3$-$C_6$-Alkinyl oder $R_{10}$ und $R_{11}$ zusammen eine gegebenenfalls durch Methyl substituierte oder Sauerstoff, Schwefel, —SO—, —$SO_2$—, —NH— oder —N($C_1$-$C_4$-Alkyl)-unterbrochene $C_4$-$C_6$-Alkylenbrücke,

$R_{12}$ $C_1$-$C_4$-Alkyl, $C_1$-$C_4$-Halogenalkyl oder Alkoxyalkyl mit höchstens 4 Kohlenstoffatomen,

$R_{13}$ Wasserstoff, $C_1$-$C_4$-Alkyl, $C_3$-$C_5$-Alkenyloxy, $C_3$-$C_5$-Alkinyloxy, $C_1$-$C_5$-Alkylthio, Phenoxy, Benzyloxy, —$NR_{10}R_{11}$ oder gegebenenfalls durch 1 bis 3 Halogenatome substituiertes $C_1$-$C_5$-Alkoxy,

$R_{14}$ $C_1$-$C_6$-Alkyl, $C_2$-$C_6$-Alkenyl oder $C_3$-$C_6$-Alkinyl und

X und Y unabhängig voneinander Sauerstoff, Schwefel, eine Sulfinyl- oder Sulfonylbrücke bedeuten, wobei $R_8$ die gleiche Bedeutung wie $R_7$ und $R_9$ die gleiche Bedeutung wie $R_5$ haben ; sowie Salze dieser Verbindungen, mit der Massgabe, dass

a) $R_1$ nur dann die angegebene Bedeutung hat, wenn $R_4$ für die Gruppe —X—$CHF_2$ steht, und dass

b) $R_4$ nur dann die angegebene Bedeutung hat, wenn $R_1$ für den substituierten Phenylkern steht und $R_5$ für die Gruppe —Y—$C_2$-$C_6$-Halogenalkenyl stehen.

Bevorzugte Untergruppen von Verbindungen der Formel I sind dadurch gekennzeichnet, dass in ihnen entweder

a) $R_5$ für eine Gruppe —Y—$C_2$-$C_6$-Halogenalkenyl oder

b) $R_4$ für eine Gruppe —X—$CHF_2$ steht.

Bevorzugt werden ferner diejenigen Verbindungen im Umfang der Formel I, worin X, Y und Z für Sauerstoff stehen und der Substituent $R_1$ eine einfach in 2-Stellung durch Halogen, Alkoxycarbonyl, Alkoxy, Alkenyloxy, Halogenalkenyloxy oder Halogenalkoxy substituierte Phenylgruppe darstellt.

Bevorzugt sind auch diejenigen Verbindungen der Formel I, worin $R_2$ für Wasserstoff steht.

Insbesondere bevorzugt sind die Verbindungen der Formel I, in denen $R_1$ für in 2-Stellung durch Fluor, Chlor, Nitro, $C_1$-$C_4$-Alkoxycarbonyl, $C_2$-$C_5$-Alkoxyalkoxy, $C_1$-$C_4$-Halogenalkoxy, $C_1$-$C_6$-Halogenalkylthio oder $C_1$-$C_4$-Halogenalkyl substituiertes Phenyl, $R_2$ für Wasserstoff, Z für Sauerstoff, $R_3$ für Wasserstoff oder $C_1$-$C_4$-Alkyl und $R_4$ für $C_1$-$C_4$-Alkyl, $C_1$-$C_4$-Alkoxy, $C_1$-$C_4$-Alkylthio, —N—di-$C_1$-$C_4$-Alkyl, oder —$SO_2$—$C_1$-$C_4$-Alkyl stehen.

Hiervon sind wiederum die Verbindungen bevorzugt, in denen $R_1$ für in 2-Stellung durch Fluor, Chlor, Nitro, Methoxycarbonyl, Methoxyäthoxy, Difluormethoxy oder Difluormethylthio substituiertes Phenyl, $R_2$ für Wasserstoff, Z für Sauerstoff, $R_3$ für Wasserstoff oder $C_1$-$C_4$-Alkyl und $R_3$ für $C_1$-$C_4$-Alkyl, $C_1$-$C_4$-Alkoxy oder $C_1$-$C_4$-Alkylthio stehen.

Weitere bevorzugte Verbindungen gehörten zur Untergruppe der Formel IX

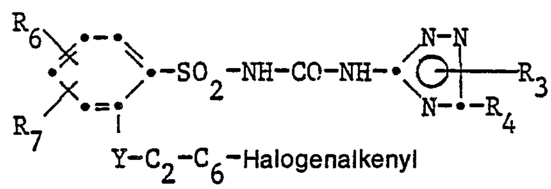

(IX)

worin $R_3$ für $C_1$-$C_4$-Alkyl oder Wasserstoff, $R_4$ für $C_1$-$C_4$-Alkoxy, $C_1$-$C_4$-Alkylthio, —N—di-$C_1$-$C_4$-Alkyl, —$SO_2$—$C_1$-$C_4$-Alkyl oder $C_1$-$C_4$-Halogenalkoxy und Y für Sauerstoff oder Schwefel stehen und $R_6$ und $R_7$ die unter Formel I gegebene Bedeutung haben.

Bevorzugt sind solche Verbindungen der Formel IX, in denen $R_6$ und $R_7$ Wasserstoff bedeuten.

Von diesen Gruppen sind insbesondere diejenigen Verbindungen von besonderem Interesse, in denen die —Y—$C_2$-$C_6$-Halogenalkenylgruppe für 1,2-Dichlorvinyloxy oder 1,2-Dichlorvinylthio steht. Vorzugsweise bedeutet dieser Substituent 1,2-Dichlorvinyloxy.

Ebenso bevorzugt sind Verbindungen der Formel I, in denen $R_1$ für in 2-Stellung durch Fluor, Chlor, Nitro, $C_1$-$C_4$-Alkoxycarbonyl, $C_2$-$C_5$-Alkoxyalkoxy, $C_1$-$C_4$-Halogenalkoxy, $C_1$-$C_4$-Halogenalkylthio, $C_1$-$C_4$-Halogenalkyl oder —Y—$C_2$-$C_6$-Halogenalkenyl substituiertes Phenyl, $R_2$ für Wasserstoff, Z für Sauerstoff, $R_3$ für Wasserstoff oder $C_1$-$C_4$-Alkyl, $R_4$ für —X—$CHF_2$ und X und Y unabhängig voneinander für Sauerstoff oder Schwefel stehen.

Die Verbindungen der Formel I können je nach Konstitution der Triazolkomponente III in den drei nachfolgenden isomeren Strukturen Ia, Ib und Ic vorliegen :

(Siehe Formel Seite 5 f.)

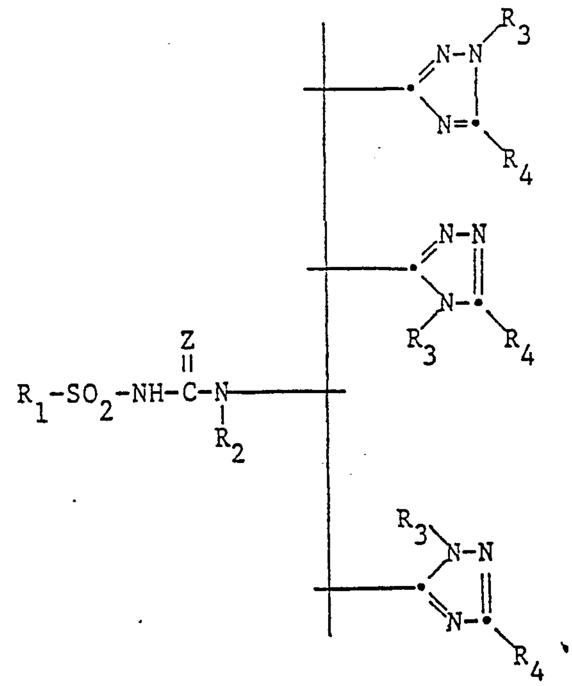

(Ia)

(Ib)

(Ic)

Die Symbole $R_1$, $R_2$, $R_3$, $R_4$ und Z haben die unter Formel I gegebenen Bedeutungen.

Die Isomeren weisen unterschiedliche herbizide Eigenschaften auf. Unter ihnen sind aufgrund ihrer vorteilhaften Wirkung diejenigen Isomeren bevorzugt, die durch die Formel Ia charakterisiert werden.

Hiervon sind wiederum die Untergruppen hervorzuheben, denen

aa) $R_1$ für in 2-Stellung durch Fluor, Chlor, Nitro, $C_1$-$C_4$-Alkoxy-carbonyl, $C_2$-$C_5$-Alkoxyalkoxy, $C_1$-$C_4$-Halogenalkoxy oder $C_1$-$C_4$-Halogenalkylthio substituiertes Phenyl, $R_2$ für Wasserstoff, Z für Sauerstoff, $R_3$ für $C_1$-$C_4$-Alkyl und $R_4$ für $C_1$-$C_4$-Alkyl, $C_1$-$C_4$-Alkoxy, $C_1$-$C_4$-Alkylthio oder —N—di-$C_1$-$C_4$-Alkyl stehen, oder

bb) $R_1$ für in 2-Stellung durch eine Gruppe —Y—$C_2$-$C_6$-Halogenalkenyl substituiertes Phenyl, $R_2$ für Wasserstoff, Z für Sauerstoff, $R_3$ für Wasserstoff oder $C_1$-$C_4$-Alkyl, $R_4$ für $C_1$-$C_4$-Alkoxy, $C_1$-$C_4$-Alkylthio, —N—di-$C_1$-$C_4$-Alkyl, —$SO_2$—$C_1$-$C_4$-Alkyl oder $C_1$-$C_4$-Halogenalkoxy und Y für Sauerstoff oder Schwefel stehen, oder

cc) $R_1$ für in 2-Stellung durch Fluor, Chlor, Nitro, $C_1$-$C_4$-Alkoxycarbonyl, $C_2$-$C_5$-Alkoxyalkoxy, $C_1$-$C_4$-Halogenalkyl oder —Y—$C_2$-$C_6$-Halogenalkenyl substituiertes Phenyl, $R_2$ für Wasserstoff, Z für Sauerstoff, $R_3$ für Wasserstoff oder $C_1$-$C_4$-Alkyl, $R_4$ für —X—$CHF_2$ und X und Y unabhängig voneinander für Sauerstoff oder Schwefel stehen ; vorzugsweise bedeutet X Sauerstoff.

In der Gruppe bb) sind die Verbindungen bevorzugt, in denen $R_1$ für 2-(1,2-Dichlorvinyloxy)-phenyl oder 2-(1,2-Dichlorvinylthio)-phenyl steht ; vorzugsweise steht $R_1$ für 2-(1,2-Dichlorvinyloxy)-phenyl.

Aufgrund ihrer besonders vorteilhaften Wirkung sind folgende Einzelsubstanzen besonders bevorzugt :

N-(2-Methoxycarbonylphenyl-sulfonyl)-N'-(1-methyl-5-difluormethoxy-1,2,4-triazol-3-yl)-harnstoff,
N[2-(1,2-Dichlorvinyloxy)-phenyl-sulfonyl]-N'-(1,5-dimethyl-1,2,4-triazol-3-yl)-harnstoff,
N-[2-(1,2-Dichlorvinyloxy)-phenyl-sulfonyl]-N'-(5-äthyl-1-methyl-1,2,4-triazol-3-yl)-harnstoff,
N-[2-(1,2-Dichlorvinyloxy)-phenyl-sulfonyl]-N'-(1-methyl-5-isopropyl-1,2,4-triazol-3-yl)-harnstoff,
N-[2-(1,2-Dichlorvinyloxy)-phenyl-sulfonyl]-N'-(1-methyl-5-cyclopropyl-1,2,4-triazol-3-yl)-harnstoff,
N-[2-(1,2-Dichlorvinyloxy)-phenyl-sulfonyl]-N'-(5-methoxy-1-methyl-1,2,4-triazol-3-yl)-harnstoff,
N-[2-(1,2-Dichlorvinyloxy)-phenyl-sulfonyl]-N'-(1-äthyl-5-methyl-1,2,4-triazol-3-yl)-harnstoff,
N-[2-(1,2-Dichlorvinyloxy)-phenyl-sulfonyl]-N'-(1,5-diäthyl-1,2,4-triazol-3-yl)-harnstoff,
N-[2-(1,2-Dichlorvinyloxy)-phenyl-sulfonyl]-N'-(5-methoxy-1-methyl-1,2,4-triazol-3-yl)-harnstoff,
N-[2-(1,2-Dichlorvinyloxy)-phenyl-sulfonyl]-N'-(1-methyl-5-difluormethoxy-1,2,4-triazol-3-yl)-harnstoff,
N-[2-(1,2-Dichlorvinyloxy)-phenyl-sulfonyl]-N'-(1,3-dimethyl-1,2,4-triazol-5-yl)-harnstoff und
N-[2-(1,2-Dichlorvinyloxy)-phenyl-sulfonyl]-N'-(5-dimethylamino-1-methyl-1,2,4-triazol-3-yl)-harnstoff.

Die Wirkstoffe der Formel I können in an sich bekannter Weise in einem reaktionsinerten, organischen Lösungsmittel oder Lösungsmittelgemisch hergestellt werden.

Man kann dabei im einzelnen folgendermassen vorgehen und Verbindungen der Formel I, gemäss Gleichung [1],

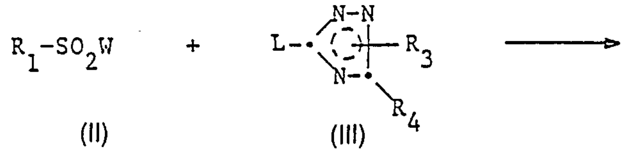 (I) [1]

(II) (III)

dadurch herstellen, dass man ein Phenylsulfonylderivat II mit einer Verbindung der Formel III umsetzt, wobei L und W für die Gruppen $—NH_2$, $—N=C=Z$ oder $—NR_2—CZ—OR$ stehen ; die Substituenten $R_1$, $R_2$, $R_3$, $R_4$, und Z die unter Formel I angegebenen Bedeutungen haben, R für einen aliphatischen oder aromatischen Rest, vorzugsweise für $C_1$-$C_4$-Alkyl, Phenyl oder Benzyl steht ; mit der Massgabe, dass die Reaktionspartner der Formeln II und III stets so gewählt werden, dass eine Aminofunktion entweder mit einer Isocyanato- bzw. Isothiocyanatofunktion oder mit der [$—NR_2—CZ—OR$]-Gruppe zur Reaktion gelangt.

Das Verfahren wird vorteilhafterweise in Gegenwart einer Base durchgeführt. In manchen Fällen kann es von Vorteil sein, wenn man unter Schutzgasatmosphäre z. B. unter Stickstoff arbeitet.

Das Herstellungsverfahren ist Bestandteil vorliegender Erfindung.

Die erhaltenen Verfahrens-Produkte der Formel I können gewünschtenfalls durch Reaktion mit Aminen, Alkalimetall- oder Erdalkalimetallhydroxiden oder quaternären Ammoniumbasen in basische, landwirtschaftlich, verwendbare Additionssalze übergeführt werden. Dieses geschieht beispielsweise durch Umsetzen mit der äquimolaren Menge Base und Verdampfen des Lösungsmittels.

Die Ausgangsverbindungen der Formel II sind aus der Literatur bekannt oder werden nach an sich bekannten Methoden hergestellt.

Die als Zwischenprodukte verwendeten Phenylsulfonamide der Formel II können aus den entsprechenden Anilin-Derivaten durch Diazotierung und Austausch der Diazogruppe mit Schwefeldioxid in Gegenwart eines Katalysators wie Kupfer-I-chlorid in Salzsäure oder Essigsäure und Umsetzen des entstandenen Phenylsulfonylchlorids mit Ammoniak erhalten werden. Entsprechende Anilin-Derivate sind bekannt oder nach bekannten Methoden herstellbar. In bestimmten Fällen, z. B. bei Vorliegen einer aktivierten Substitutionsposition, ist eine direkte Sulfochlorierung des Phenylkerns möglich, die durch Umsetzung mit einem Ueberschuss Chlorschwefelsäure zu dem entsprechenden Phenylsulfonylchlorid führt. Entsprechende aktivierte Benzolderivate sind bekannt.

Die Isocyanate der Formeln II und III können durch Umsetzung der zugrundeliegenden Amino-Verbindungen mit Phosgen in Anwesenheit von Butylisocyanat in einem chlorierten Kohlenwasserstoff als Lösungsmittel, bei Rückflusstemperatur gehalten werden. Siehe dazu « Neuere Methoden der präparativen organischen Chemie », Band VI, 211-229, Verlag Chemie, Weinheim, 1970. Bei Verwendung von Thiophosgen gelangt man auf analoge Weise zu Isothiocyanaten II und III.

Die Isothiocyanate der Formel II werden darüberhinaus auch durch Behandlung der Sulfonamide II mit Schwefelkohlenstoff und Kaliumhydroxid und anschliessender Umsetzung des Dikaliumsalzes mit Phosgen erhalten, siehe Arch. Pharm. 299, 174 (1966).

Verbindungen der Formeln II und III, worin L und W für die Gruppe $—NR_2—CZ—OR$ steht, können aus den zugrundeliegenden Aminoverbindungen durch Reaktion mit einem Halogen(thio)kohlensäureester der Formel IV

$$Hal - \overset{\overset{\displaystyle Z}{\|}}{C} - OR$$

hergestellt werden ; hierbei steht Hal für ein Halogen, vorzugsweise Chlor oder Brom, Z steht für Sauerstoff oder Schwefel und R für einen aliphatischen oder aromatischen Rest, vorzugsweise für $C_1$-$C_4$-Alkyl, Phenyl oder Benzyl. Diese Reaktion wird bevorzugt in Gegenwart einer Base durchgeführt.

Die Umsetzung zu Verbindungen der Formel I werden vorteilhafterweise in aprotischen, inerten organischen Lösungsmittel vorgenommen. Solche Lösungsmittel sind Kohlenwasserstoffe wie Benzol, Toluol, Xylol oder Cyclohexan, chlorierte Kohlenwasserstoffe wie Methylenchlorid, Chloroform, Tetrachlorkohlenstoff, oder Chlorbenzol, Aether wie Diäthyläther, Aethylenglykoldimethyläther, Diäthylenglykoldimethyläther, Tetrahydrofuran oder Dioxan, Nitrile wie Acetonitril oder Propionitril, Amide wie Dimethylformamid, Diäthylformamid oder N-Methylpyrrolidinon. Die Reaktionstemperaturen liegen vorzugsweise zwischen — 20° und + 120 °C. Die Umsetzungen der Kupplungsverfahren verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base oder Isocyanat als Reaktionskatalysator verkürzt werden. Als Basen sind insbesondere tertiäre Amine wie Trimethylamin, Triäthylamin, Chinuclidin, 1,4-Diazabicyclo-(2,2,2)-octan. 1,5-Diazabicyclo(4,3,0)non-5-en oder 1,5-Diazabicyclo(5,4,0) undec-7-en geeignet.

Die Endprodukte können durch Einengen und/oder Verdampfen des Lösungsmittels isoliert und durch Umkristallisieren oder Zerreiben des festen Rückstands in Lösungsmitteln, in denen sie sich nicht gut lösen, wie Aether, aromatischen Kohlenwasserstoffen oder chlorierten Kohlenwasserstoffen, gereinigt werden.

Die Wirkstoffe der Formel I sind stabile Verbindungen. Ihre Handhabung bedarf keiner vorsorglichen Massnahmen. Sie sind in den üblichen organischen Lösungsmitteln relativ gut, in Wasser hingegen wenig löslich. Sie können durch Zugabe von Wasser zur Reaktionslösung leicht ausgefällt werden. Ihre Formulierung als flüssige herbizide oder pflanzenbakterizide Mittel gelingt mit Hilfe üblicher Lösungsvermittler und/oder Dispergiermittel.

Die Verbindungen der Formel III sind zum Teil neu. Je nach der isomeren Struktur der substituierten 3-Amino-1,2,4-triazole sind diese Verbindungen nach an sich bekannten Verfahren erhältlich oder sie werden nach einem neuen Verfahren hergestellt.

Die Verbindungen der Formel IIIa

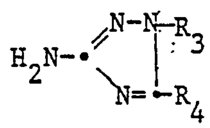

(IIIa)

worin $R_3$ Wasserstoff oder unverzweigtes $C_1$-$C_6$-Alkyl bedeutet und $R_4$ die unter Formel I gegebene Bedeutung hat, werden durch Umsetzung einer N-Cyanimino-Verbindung der Formel V

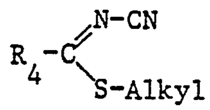

(V)

worin $R_4$ die unter Formel I gegebene Bedeutung hat und Alkyl für eine $C_1$-$C_6$-Alkylgruppe steht, mit einem Hydrazin der Formel VI

$$H_2N—NH—R_3 \qquad (VI)$$

worin $R_3$ die unter Formel IIIa gegebene Bedeutung hat.

· Die Umsetzungen der Verbindungen der Formeln V und VI werden mit Vorteil in einem inerten organischen Lösungsmittel bei Temperaturen zwischen —30 °C und + 120 °C, vorzugsweise zwischen —10° und + 90 °C, durchgeführt. Als Lösungsmittel eignen sich beispielsweise Alkohole wie Methanol, Aethanol oder Isopropanol, Aether wie Diäthyläther, Athylenglykoldimethyläther, Tetrahydrofuran oder Dioxan, Halogenkohlenwasserstoffe wie Methylenchlorid, Chloroform, Tetrachlorkohlenstoff, Trichloräthan oder Trichloräthylen, Acetonitril, Toluol oder Benzol.

Solche Umsetzung sind in der Literatur [J. Org. Chem. 39, 1522 (1974)] beschrieben. Es wurde dabei den Produkten die isomere Struktur IIIc zugeschrieben.

Die Ausgangsmaterialien der Formeln V und VI sind bekannt oder nach an sich bekannten Verfahren erhältlich, siehe J.C.S. Chem. Comm., 1974, 350, Fussnote 1., J. Org. Chem. 39, 1522 (1974) und Archiv der Pharmazie, 303, 625 (1970).

Verbindungen der Formel IIIa, in denen $R_3$ $C_1$-$C_6$-Alkyl und $R_4$ für $C_1$-$C_4$-Alkoxy, $C_1$-$C_4$-Alkylthio, N—di-$C_1$-$C_4$-Alkylamino, oder Alkoxyalkoxy mit höchstens 4 Kohlenstoffatomen stehen, erhält man nach einem neuen Verfahren, indem man ein Iminokohlensäurehydrazid der Formel VII

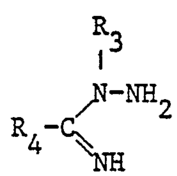

(VII)

worin $R_3$ $C_1$-$C_6$-Alkyl und $R_4$ für $C_1$-$C_4$-Alkoxy, $C_1$-$C_4$-Alkylthio, N—di-$C_1$-$C_4$-Alkylamino oder Alkoxyalkoxy mit höchstens 4 Kohlenstoffatomen steht, mit Chlorcyan in Gegenwart einer Base umsetzt. Dieses Verfahren ist neu.

Diese Umsetzung des Iminokohlensäurehydrazids der Formel VII mit Chlorcyan wird zweckmässigerweise in einem inerten organischen Lösungsmittel bei Temperaturen zwischen —20° und + 70 °C, vorzugsweise zwischen 0° und + 40 °C, durchgeführt. Als Base dienen dabei vorzugsweise organische Basen wie tertiäre Amine, z. B. Trimethylamin, Triäthylamin, Pyridin, Chinolin, Isochinolin, Chinuclidin oder Diazobicyclooctan, insbesondere aber Triäthylamin.

Ebenfalls neu ist die Herstellung der Verbindungen der Formel VII aus einem 1-Alkyl-1-cyan-hydrazin und einer Verbindung $R_4$—H in alkoholischer Lösung.

Die als Zwischenprodukte verwendbaren 1-Alkyl-1-cyan-hydrazine sind nach einem in der DE-A-25 25 852 beschriebenen Verfahren erhältlich.

Die Verbindungen der Formel IIIb

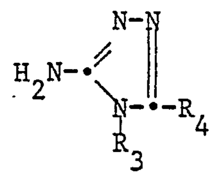

(IIIb)

sind aus der Literatur bekannt oder können analog zu bekannten Verfahren hergestellt werden. Solche Verfahren sind in Chem. Ber. 97, 396 (1964) beschrieben.

Die Verbindungen der Formel IIIc

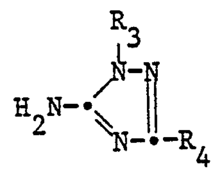

(IIIc)

worin $R_3$ und $R_4$ die unter Formel I gegebene Bedeutung haben, mit der Massgabe, dass $R_4$ nicht ein über ein Schwefelatom gebundener Rest sein darf, werden erhalten, indem man eine N-Cyanimino-verbindung der Formel VIII

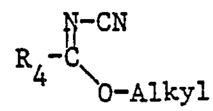

(VIII)

mit einem Hydrazin der Formel VI umsetzt, wobei $R_3$ und $R_4$ die unter Formel IIIc gegebene Bedeutung haben und Alkyl für $C_1$-$C_6$-Alkyl steht. Solche Umsetzungen sind in J. Org. Chem. 39, 1522 (1974) beschrieben.

Diejenigen Verbindungen der Formel IIIc, in denen $R_4$ die unter Formel I gegebene Bedeutung hat und $R_3$ für Wasserstoff oder eine verzweigte Alkylgruppe stehen, können erhalten werden, indem man eine N-Cyanimino-Verbindung der Formel V mit einem Hydrazin der Formel VI wobei $R_3$ und $R_4$ die oben angegebene Bedeutung haben.

Verbindungen der Formel III, in denen $R_4$ für Difluormethoxy oder Difluormethylthio steht, können auch durch Verätherung mit Difluorchlormethan in Gegenwart von Basen aus entsprechenden Hydroxyl- oder Mercaptoverbindungen erhalten werden. Beispielsweise erhält man so 3-Amino-5-difluormethoxy-1-methyl-1,2,4-triazol durch Umsetzen des aus Chem. Ber. 755 (1969) bekannten 3-Amino-5-hydroxy-1-methyl-1,2,4-triazols mit Difluorchlormethan in Gegenwart von wässriger Natronlauge.

Die Ausgangsmaterialien der Formel VIII sind bekannt oder werden nach an sich bekannten Methoden hergestellt, siehe J. Org. Chem. 28, 1816 (1963) und EP-A-14 064 (1980).

Die neuen Verbindungen der Formel III, die der Formel IIId

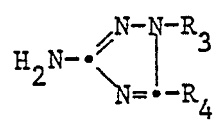

(IIId)

entsprechen, worin $R_3$ für $C_1$-$C_6$-Alkyl steht und $R_4$ für $-X-CHF_2$ steht, wurden speziell als Zwischenprodukte für die Synthese der pflanzenwuchsregulierenden Wirkstoffe der Formel I entwickelt.

Es wurde nunmehr überraschend gefunden, dass die erfindungsgemässen Wirkstoffe der Formel I gute selektiv-herbizide Eigenschaften aufweisen. Dabei kann teilweise auch beobachtet werden, dass selbst Problemunkräuter geschädigt werden, denen mit bisher bekannten Herbiziden nicht beizukommen war.

Weiter wurde überraschend festgestellt, dass die erfindungsgemässen Wirkstoffe der Unterformel IX besonders wertvolle pflanzenbakterizide Eigenschaften besitzen. Dadurch können phytopathogene Bakterien in Kulturpflanzenbeständen bekämpft werden, ohne dass bei den behandelten Kulturpflanzen Schädigungen beobachtet werden können.

Verbindungen der Formel I zeigen translozierende Eigenschaften, d. h. sie werden an der behandelten Stelle der Pflanze (Blatt, Stiel, Wurzel etc.) aufgenommen und an andere Stellen transportiert, wo sie ihre Wirkung entfalten. Sie folgen hierbei nicht nur der Richtung des Nährstofftransportes von den Wurzeln in die Blätter, sondern gelangen auch umgekehrt von den Blättern zu den Wurzeln. So gelingt es mit Hilfe dieser translozierenden Eigenschaft z. B. durch eine Oberflächenbehandlung perennierende Unkräuter bis in die Wurzeln zu vernichten. Darüberhinaus wirken die Wirkstoffe der

Formel I im Vergleich zu herkömmlichen Herbiziden bereits in sehr geringen Aufwandmengen.

Die Wirkstoffe der Formel IX sind auch gegen phytopathogene Bakterien aktiv wie z. B. Pseudomonas spp. Xanthomonas spp. sowie Erwinia und Corynebacterium. Ueberdies wirken die Verbindungen zum Teil systemisch. Sie können ferner als Beizmittel zur Behandlung von Saatgut (Früchte, Knollen, Körner) und Pflanzenstecklingen zum Schutz von Infektionen sowie gegen im Erdboden auftretende phytopathogene Mikroben eingesetzt werden. Die vorliegende Erfindung betrifft somit ferner die Verwendung der Verbindungen der Formel IX zur Bekämpfung von phytopathogenen Bakterien.

Die Verbindungen der Formel IX besitzen ein für die praktischen Bedürfnisse sehr günstiges Bakterizid-Spektrum zum Schutze von Kulturpflanzen, ohne diese durch unerwünschte Nebenwirkungen nachteilig zu beeinflussen. Kulturpflanzen sind im Rahmen vorliegender Erfindung beispielsweise Getreide, Mais, Reis und Gemüse.

Mit Verbindungen der Formel IX können an Pflanzen oder Pflanzenteilen (Früchte, Blüten, Laubwerk, Stengel, Knollen, Wurzeln) dieser und verwandter Nutzkulturen die auftretenden Bakterien eingedämmt oder vernichtet werden, wobei auch später zuwachsende Pflanzenteile von derartigen Bakterien verschont bleiben.

Werden die Aufwandmengen der erfindungsgemässen Wirkstoffe weiter gesenkt, so treten vor allem die wachstumsregulierenden Eigenschaften in den Vordergrund, dabei greifen die Verbindungen der Formel I gezielt in den Metabolismus der Pflanzen ein und führen insbesondere zu einer Reduktion des vegetativen Wachstums, oft zugunsten des generativen Wachstums. Dieser gezielte Eingriff in die physiologischen Vorgänge der Pflanzenentwicklung macht die Wirkstoffe der Formel I für verschiedene Zwecke verwendbar, insbesondere für solche, die mit der Arbeitseinsparung bei Massnahmen an Pflanzenkulturen oder der Ertragssteigerung im Zusammenhang stehen.

Hierin offenbarte Verbindungen der Formel I können als Herbizide und/oder als Wachstumsregulatoren eingesetzt werden. Nach bisherigen Erfahrungen gilt für die Applikation von Wachstumsregulatoren, dass die Aktivsubstanzen eine oder mehrere unterschiedliche Wirkungen bei den Pflanzen hervorrufen können. Diese verschiedenartigen Wirkungen hängen im wesentlichen vom Zeitpunkt der Anwendung, d. h. vom Entwicklungsstadium des Samens oder der Pflanze, der Art der Applikation sowie insbesondere von den angewendeten Konzentrationen ab. Derartige Effekte sind aber wiederum je nach Pflanzenart verschieden. Die Applikation von Verbindungen der Formel I eröffnet somit die Möglichkeit, das Wachstum von Pflanzen in gewünschter Weise zu beeinflussen.

Mit den neuen Wirkstoffen der Formel I bzw. mit den neuen Mitteln wird das vegative Pflanzenwachstum gedämpft. Durch diese Beeinflussung des Wachstums können die erfindungsgemäss verwendeten Wirkstoffe die Ernteausbeute von Pflanzen wesentlich erhöhen. So erfahren etwa Sojapflanzen und ander Leguminosen wie Bohnen, Erbsen oder Linsen eine Reduktion des vegativen zugunsten des generativen Wachstums, wodurch eine unmittelbare Ertragssteigerung erzielt wird. Auch bei weiteren Pflanzenarten, z. B. bei Reben, Getreidesorten, Gräsern und Zierpflanzen wird das vegative Wachstum in gewünschter Weise gehemmt. Daneben beobachtet man bei den behandelten Pflanzen eine deutliche Stärkung des Stützgewebes.

Eine im Vordergrund stehende Art der Pflanzenbeeinflussung beruht auf der besonderen Eigenschaft der Verbindungen der Formel I, bei bestimmten Pflanzen, insbesondere bei Getreidekulturen, eine gezielte Wuchshemmung hervorzurufen, die bei unveränderter Ertragsleistung die Knickfestigkeit der Pflanzen wesentlich erhöht. Daraus ergibt sich eine wirtschaftlich sehr interessante Methode des Schutzes von Pflanzenkulturen vor Lagerung durch Sturm oder unwetter. Darüberhinaus gestattet eine gezielte Hemmung des vegativen Wachstums bei vielen Kulturpflanzen eine dichtere Anpflanzung der Kulturen ; dies führt bei gleichem Fruchtansatz und gleicher Anbauglâche zu einer deutlichen Ertragssteigerung. Der Einsatz von Wuchshemmern bewirkt auch eine effektivere Nutzung der Nährstoffe, die nunmehr in verstärktem Masse der Blüten und Fruchtbildung zutute kommen. Auf diese Weise lassen sich höhere Ernteerträge bei gleichzeitig kleinerem Abfallanteil an vegetativen Pflanzenresten [z. B. Stroh, Kartoffelkraut, Rübenblätter] erzielen.

Hervorzuheben ist auch die Möglichkeit, mit den erfindungsgemässen Stoffen bzw. Mitteln bei verschiedenen Pflanzenarten, insbesondere bei Tabakpflanzen eine Hemmung des unerwünschten Geiztriebwuchses zu erreichen, wenn der Haupttrieb kurz vor der Blüte zum Zwecke des erstrebten Wuchsvergrösserung der Blätter abgeschnitten worden ist.

Die Erfindung betrifft somit eine herbizide, bakterizide und/oder wachstumsregulierende Verwendung der Verbindungen der Formel I. Eingeschlossen ist auch ein Verfahren zur pre- und postemergenten Unkrautbekämpfung, zur Hemmung des Pflanzenwuchses von monokotylen und dikotylen Pflanzen, insbesondere von Gräsern, Getreidesorten und Tabakgeiztrieben, sowie zur Steigerung des Ernteertrages bei Leguminosen sowie zur Bekämpfung von phytopathogenen Bakterien bei Kulturpflanzen.

Die Verbindungen der Formel I werden in unveränderter Form oder vorzugsweise als Mittel zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den

gegebenen Verhältnissen entsprechend gewählt.

Die Formulierungen, d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen : Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl ; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z. B. die Na- und K-Salze der Oel- oder Stearinsäure, oder von natürlichen Fettsäuregemischen, die z. B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Liginsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z. B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylglykoläthergruppen und 10 bis 100 Propylenglykoläthergruppen enthaltenden Polyäthylenoxiaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylenpolyäthylenoxid-addukte, Tributylphenoxypolyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quaternäre Ammoniumsalze, welches als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z. B. das Stearyltrimethylammoniumchlorid oder das Benzylid(2-chloräthyl)-äthylammoniumbromid.

Die in der Formulierungstechnik gebräuchlichen Tenside sind u. a. in folgenden Publikationen beschrieben :

« Mc Cutcheon's Detergents and Emulsifiers Annual» MC

Publishing Corp., Ridgewood, New Jersey, 1979.
Sisley and Wood, « Encyclopedia of Surface Active Agents »
Chemical Publishing Co., Inc. New York, 1964.

Die pestiziden Zubereitungen enthalten in der Regel 0,1 bis 95 %, insbesondere 0,1 bis 80 %, Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen : (% = Gewichtsprozent).

| | |
|---|---|
| Emulgierbare Konzentrate | |
| Aktiver Wirkstoff : | 1 bis 20 %, bevorzugt 5 bis 10 % |
| oberflächenaktives Mittel : | 5 bis 30 %, vorzugsweise 10 bis 20 % |
| flüssiges Trägermittel : | 50 bis 94 %, vorzugsweise 70 bis 85 %. |
| Stäube | |
| Aktiver Wirkstoff : | 0,1 bis 10 %, vorzugsweise 0,1 bis 1 % |
| festes Trägermittel : | 99,9 bis 90 %, vorzugsweise 99,9 bis 99 %. |
| Suspension-Konzentrate | |
| Aktiver Wirkstoff : | 5 bis 75 %, vorzugsweise 10 bis 50 % |
| Wasser : | 94 bis 25 %, vorzugsweise 90 bis 30 % |
| oberflächenaktives Mittel : | 1 bis 40 %, vorzugsweise 2 bis 30 %. |
| Benetzbare Pulver | |
| Aktiver Wirkstoff : | 0,5 bis 90 %, vorzugsweise 1 bis 80 % |
| oberflächenaktives Mittel : | 0,5 bis 20 %, vorzugsweise 1 bis 15 % |
| festes Trägermittel : | 5 bis 95 %, vorzugsweise 15 bis 90 %. |
| Granulate | |
| Aktiver Wirkstoff : | 0,5 bis 30 %, vorzugsweise 3 bis 15 % |
| festes Trägermittel : | 99,5 bis 70 %, vorzugsweise 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,01 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zu Erzielung spezieller Effekte enthalten.

Die Erfindung umfasst auch derartige landwirtschaftlich verwendbare Mittel, die als mindestens eine Aktivsubstanz eine Verbindung der Formel I enthalten.

Darüberhinaus schliesst die vorliegende Erfindung auch die Herstellung solcher Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

In den nachfolgenden Beispielen sind Temperaturen in Celsiusgraden angegeben, Prozentangaben und Teile beziehen sich auf das Gewicht.

## Herstellungsbeispiele

### Beispiel 1 : Herstellung des Zwischenprodukts

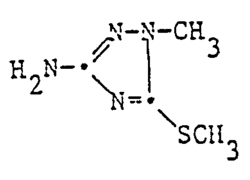

3-Amino-1-methyl-5-methylthio-1,2,4-triazol

Zur Lösung von 14,6 g N-Cyanimido-dithiokohlensäure-dimethylester in 50 ml Aethanol lässt man bei 0 °C eine Lösung von 5,0 g Methylhydrazin in 15 ml Aethanol langsam zutropfen. Anschliessend wird die Lösung für eine weitere Stunde bei der gleichen Temperatur weiter gerührt und dann eingedampft. Der feste Rückstand wird mit Aether gewaschen und getrocknet.

Ausbeute 13,2 g (92 % d. Th.), Smp. 102 °C.
$^1$H-NMR($CDCl_3$) : δ = 2,60 (s, 3H ; $SCH_3$) ; 3,58 (s, 3H ; $NCH_3$) ; 4,65 (s, 2H ; $NH_2$) ppm.
Analyse : $C_4H_8N_4S$ (144.20)
Ber. : C 33,32 H 5,60 N 38,86 S 22,24
Gef. : C 33,2 H 5,5 N 39,2 S 22,3

Beispiel 2 : Herstellung des Zwischenprodukts

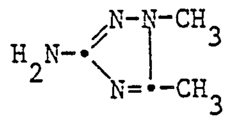

3-Amino-1,5-dimethyl-1,2,4,-triazol

a) N-Cyano-thioacetimidsäureäthylester

Zur Lösung von 35 g Chlorcyan in 200 ml Aceton und 200 ml Chloroform werden 70 g Thioacetimidsäureäthylester zugesetzt. In diese Mischung lässt man bei 0 °C 154 ml Triäthylamin zutropfen und rührt das Reaktionsgemisch bei der gleichen Temperatur für 5 Stunden. Nach dem Abtrennen des Niederschlags wird die Lösung eingedampft und der oelige Rückstand im Vakuum destilliert.
Ausbeute : 58,2 g (91 % d. Th.), Sdp. 55 °C/0,09 mbar.
$^1$H-NMR($CDCl_3$) δ = 1,30 (t, 3H ; $CH_3$) ; 2,53 (s, 3H ; $CH_3$) ; 3,05 (q, 2H ; $CH_2$) ppm.
Analyse : $C_5H_8N_2S$ (128,19)
berechnet : C 46,85 H 6,29 N 21,86
gefunden : C 47,0 H 6,4 N 21,6

b) Zur Lösung von 12,8 N-Cyano-thioacetimidsäureäthylester in 50 ml Chloroform lässt man bei 0 °C 5,0 g Methylhydrazin im 50 ml Chloroform zutropfen. Anschliessend wird die Lösung für 16 Stunden zum Rückfluss erhitzt und dann eingedampft. Der feste Rückstand wird mit Aether gewaschen und getrocknet.
Ausbeute : 10,3 g (92 % d. Th.), Smp. 210-213 °C.
$^1$H-NMR ($CDCl_3$) : δ = 2,32 (s, 3H ; C—$CH_3$) ; 3,62 (s, 3H ; N—$CH_3$) ; 4,10 (s, 2H ; $NH_2$) ppm.
Analyse : $C_4H_8M_4$ (112,14)
berechnet : C 42,85 H 7,20 N 49,97
gefunden : C 42,9 H 7,0 N 50,1

Beispiel 3 : Herstellung des Zwischenprodukts

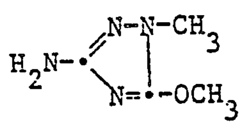

3-Amino-5-methoxy-1-methyl-1,2,4-triazol

Zur Lösung von 13,0 g N-Cyano-imidothiokohlensäure-O,S-dimethylester in 100 ml Aethanol lässt man bei 0 °C 5,0 g Methylhydrazin zutropfen und rührt das Gemisch für 18 Stunden bei der gleichen Temperatur. Nach dem Abdampfen des Lösungsmittels erhält man einen festen Rückstand, der mit Aether gewaschen, getrocknet und aus Acetonitril umkristallisiert wird.
Ausbeute : 9,9 g (77 % d. Th.), Smp. 173-175 °C.
$^1$H-NMR ($CDCl_3$) : δ = 3,33 (s, 3H ; N—$CH_3$) ; 3,94 (s, 3H ; O—$CH_3$) ; 4,60 (s, 2H ; $NH_2$).
Analyse : $C_4H_8N_4O$ (128,14)
berechnet : C 37,50 H 6,30 N 43,73
gefunden : C 37,0 H 6,1 N 44,0

Beispiel 4 : Herstellung des Zwischenprodukts

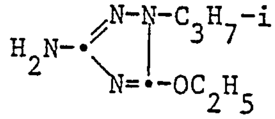

3-Amino-5-äthoxy-1-isopropyl-1,2,4-triazol

a) O-Aethyl-iminokohlensäure-1-isopropylhydrazid $[H_5C_2O-\overset{NH}{\overset{\|}{C}}-N(C_3H_7i)-NH_2]$

In eine Lösung von 0,5 g Natrium in 58,5 ml Aethanol lässt man bei 70 °C 100 g 1-Cyano-1-isopropylhydrazin eintropfen und rührt das Gemisch bei 90 °C für 2 Stunden. Durch Fraktionierung der Reaktionsmischung im Vakuum erhält man 119 g (82 % d.Th.) des Produktes, Sdp. 105°/40 mbar.

$^1$H-NMR ($CDCl_3$) : δ = 1,95 (d, 6H ; Isopropyl-$CH_3$) ; 1,30 (t, 3H ; Aethyl-$CH_3$) ; 3,4 (s, 2H ; $NH_2$) ; 4,0-4,5 (m, 3H ; CH und $CH_2$) ; 5,8 (s, 1H ; NH) ppm.

b) Zur Lösung von 14,5 g O-Aethyl-iminokohlensäure-1-isopropylhydrazid und 14 ml Triäthylamin in 50 ml Aceton lässt man bei 0 °C 8,0 g Chlorcyan in 30 ml Aceton zutropfen und rührt das Gemisch bei der gleichen Temperatur für weitere 2 Stunden. Die Reaktionslösung wird eingedampft und der ölige Rückstand im Vakuum destilliert.

Ausbeute : 13,0 g (76 % d.Th.) Sdp. 102°/0,4 mbar, Smp. 65-66 °C.

$^1$H-NMR($CDCl_3$) : δ =1,36 (d, 6H ; Isopropyl-$CH_3$) ; 1,40 (t, 3H ; Aethyl-$CH_3$) ; 4,3 (s, 2H ; $NH_2$) ; 4,35 (m, 1H ; CH) ; 4,40 (q, 2H ; $CH_2$) ppm.

Analyse : $C_7H_{14}N_4O$ (170,22)
berechnet : C 49,40 H 8,29 N 32,92
gefunden : C 49,60 H 8,5 N 32,9

Beispiel 5 : Herstellung des Zwischenprodukts

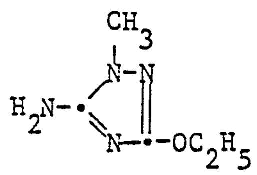

5-Amino-3-äthoxy-1-methyl-1,2,4-triazol

a) N-Cyano-iminokohlensäurediäthylester

In eine Lösung von 58,5 g Iminokohlensäurediäthylester und 37,0 Chlorcyan in 350 ml Aceton lässt man bei 0 °C 70 ml Triäthylamin eintropfen und rührt die Reaktionsmischung für 2 Stunden bei der gleichen Temperatur, filtriert den Niederschlag ab und dampft das Lösungsmittel ab. Der öelige Rückstand wird im Vakuum destilliert.

Ausbeute : 60,0 g (80 % d. Th), Sdp. 90°/0,08 mbar.

$^1$H-NMR($CDCl_3$) : δ = 1,50 (t, 6H ; $CH_3$) ; 4,40 (q, 4H ; $CH_2$) ppm.

Analyse : $C_6H_{10}N_2O_2$ (142,16)
berechnet : C 50,70 H 7,09 N 19,71
gefunden : C 50,7 H 7,0 N 19,6 .

b) Zur Lösung von 14,2 g N-Cyano-iminokohlensäurediäthylester in 100 ml Aethanol lässt man bei 0 °C 5,0 g Methylhydrazin eintropfen und rührt die Lösung für 5 Stunden bei der gleichen Temperatur. Das Produkt wird durch Einengen der Reaktionsmischung und Kristallisation aus Acetonitril gewonnen.

Ausbeute : 10,7 g (75 % d. Th.), Smp. 105-106 °C.

$^1$H-NMR($CDCl_3$) : δ = 1,33 (t, 3H ; Aethyl-$CH_3$) ; 3,46 (s, 3H ; N—$CH_3$) ; 4,18 (q, 2H ; $CH_2$) ; 5,55 (s, 2H ; $NH_2$) ppm.

Analyse : $C_5H_{10}N_4O$ (142,16)
berechnet : C 42,25 H 7,09 N 39,41
gefunden : C 42,6 H 6,8 N 39,0

Beispiel 6 : Herstellung von

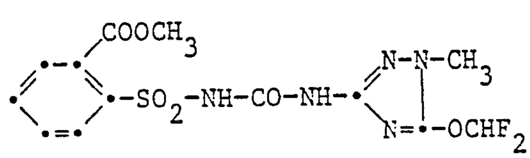

N-(2-Methoxycarbonyl-phenylsulfonyl)-N'-(5-difluormethoxy-1-methyl-1,2,4-triazol-3-yl)-harnstoff

a) 3-Amino-5-difluormethoxy-1-methyl-1,2,4-triazol

In ein Gemisch aus 10,0 g 3-Amino-5-hydroxy-1-methyl-1,2,4-triazol, 50 ml 30 %iger wässriger Natronlauge und 50 ml Dioxan leitet man bei 75 °C für 30 Minuten Difluorchlormethan-Gas ein. Nach Abkühlen des Reaktionsgemisches wird die organische Phase abgetrennt, getrocknet, eingedampft und durch Chromatographie an Kieselgel gereinigt. Man erhält so 3-Amino-5-difluormethoxy-1-methyl-1,2,4-triazol vom Schmelzpunkt 138-139 °C.

b) Eine Lösung von 1,1 g 2-Methoxycarbonyl-phenylsulfonylisocyanat und 0,75 g 3-Amino-5-difluormethoxy-1-methyl-1,2,4-triazol in 20 ml Dioxan wird für 3 Stunden auf 70 °C erhitzt. Anschliessend wird die Reaktionsmischung eingedampft und der Rückstand aus Aether kristallisiert. Man erhält so N-(2-Methoxycarbonyl-phenylsulfonyl)-N'-(5-difluormethoxy-1-methyl-1,2,4-triazol-3-yl)-harnstoff vom Schmelzpunkt 160-162 °C.

Beispiel 7 : Herstellung von

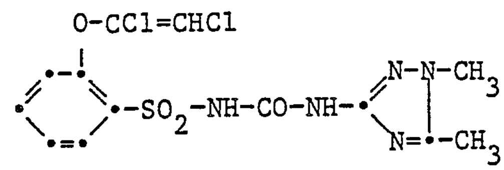

N-[2-(1,2-Dichlorvinyloxy)-phenylsulfonyl]-N'-(1,5-dimethyl-1,2,4-triazol-3-yl)-harnstoff

Zu einer Suspension von 11,2 g 3-Amino-1,5-dimethyl-1,2,4-triazol in 200 ml Dioxan lässt man bei 20 bis 25 °C 30 g 2-(1,2-Dichlorvinyloxy)-phenylsulfonylisocyanat zutropfen. Dabei tritt eine schwach exotherme Reaktion auf. Anschliessend wird die Reaktionsmischung für 18 Stunden bei 20-25° gerührt. Das ausgefallene Produkt wird abgetrennt und aus Acetonitril umkristallisiert.

Ausbeute : 26,8 g (66 % d. Th.)

N-[2-(1,2-Dichlorvinyloxy)-phenylsulfonyl]-N'-(1,5-dimethyl-1,2,4-triazol-3-yl)-harnstoff, Smp. 202-204 °C.

Analyse : $C_{13}H_{13}Cl_2N_5O_4S$ (406,24)

berechnet : C 38,44 % H 3,23 % N 17,24 % S 7,89 % Cl 17,46 %

gefunden : C 39,9 % H 3,5 % N 17,4 % S 7,6 % Cl 17,3 %.

Auf analoge Weise lassen sich auch die in den folgenden Tabellen aufgeführten Verbindungen herstellen.

Tabelle 1 : Verbindungen der Formel Ia

| Verb. Nr. | $R_1$ | $R_2$ | Z | $R_3$ | $R_4$ | Smp. [°C] |
|---|---|---|---|---|---|---|
| 1.1 | $2-COOCH_3-C_6H_4-$ | H | O | $CH_3$ | $OCHF_2$ | 160-162 |
| 1.2 | $2-COOC_2H_5-C_6H_4-$ | H | O | $CH_3$ | $OCHF_2$ | |
| 1.3 | $2-COOCH_3-C_6H_4-$ | H | O | $C_2H_5$ | $OCHF_2$ | |
| 1.4 | $2-COOCH_3-C_6H_4-$ | H | O | $C_3H_7-i$ | $OCHF_2$ | |
| 1.5 | $2-NO_2-C_6H_4-$ | H | O | $CH_3$ | $OCHF_2$ | 179-180 |
| 1.6 | $2-OCHF_2-C_6H_4-$ | H | O | $CH_3$ | $OCHF_2$ | |
| 1.7 | $2-OCF_3-C_6H_4-$ | H | O | $CH_3$ | $OCHF_2$ | |
| 1.8 | $2-CF_3-C_6H_4-$ | H | O | $CH_3$ | $OCHF_2$ | |
| 1.9 | $2-COOCH_3-C_6H_4-$ | H | O | $CH_3$ | $SCHF_2$ | |
| 1.10 | $2-Cl-C_6H_4-$ | H | O | $CH_3$ | $OCHF_2$ | |

Tabelle 1 (Fortsetzung)

| Verb. Nr. | $R_1$ | $R_2$ | Z | $R_3$ | $R_4$ | Smp. [°C] |
|---|---|---|---|---|---|---|
| 1.11 | $2-Br-C_6H_4-$ | H | O | $CH_3$ | $OCHF_2$ | |
| 1.12 | $2-SO_2N(CH_3)_2-C_6H_4-$ | H | O | $CH_3$ | $OCHF_2$ | |
| 1.13 | $2-OC_2H_5-C_6H_4-$ | H | O | $CH_3$ | $OCHF_2$ | |
| 1.14 | 1-Naphthyl | H | O | $CH_3$ | $OCHF_2$ | |
| 1.15 | $2-S-CCl=CHCl-C_6H_4-$ | H | O | $CH_3$ | $CH_3$ | |
| 1.16 | $2-S-CCl=CHCl-C_6H_4-$ | H | O | $CH_3$ | $C_2H_5$ | |
| 1.17 | $2-S-CCl=CHCl-C_6H_4-$ | H | O | $CH_3$ | $OCH_3$ | |
| 1.18 | $2-S-CCl=CHCl-C_6H_4-$ | H | O | $CH_3$ | Cycl. $C_3H_5$ | |
| 1.19 | $2-S-CCl=CHCl-C_6H_4-$ | H | O | $C_2H_5$ | $CH_3$ | |
| 1.20 | $2-S-CCl=CHCl-C_6H_4-$ | H | O | $CH_3$ | $SCH_3$ | |
| 1.21 | $2-O-CH_2-CCl=CHCl-C_6H_4-$ | H | O | $CH_3$ | $CH_3$ | |
| 1.22 | $2-O-CH_2-CCl=CHCl-C_6H_4-$ | H | O | $CH_3$ | $OCH_3$ | |
| 1.23 | $2-O-CH_2-CCl=CHCl-C_6H_4-$ | H | O | $CH_3$ | $SCH_3$ | |
| 1.24 | $2-O-CH_2-CCl=CHCl-C_6H_4-$ | H | O | $CH_3$ | $C_2H_5$ | |
| 1.25 | $2-S-CH_2-CCl=CHCl-C_6H_4-$ | H | O | $CH_3$ | $CH_3$ | |
| 1.26 | $2-S-CH_2-CCl=CHCl-C_6H_4-$ | H | O | $CH_3$ | $C_2H_5$ | |
| 1.27 | $2-S-CH_2-CCl=CHCl-C_6H_4-$ | H | O | $CH_3$ | $OCH_3$ | |
| 1.28 | $2-S-CH_2-CCl=CHCl-C_6H_4-$ | H | O | $CH_3$ | $SCH_3$ | |

Tabelle 2: Verbindungen der Formel Ib

| Verb. Nr. | $R_1$ | $R_2$ | Z | $R_3$ | $R_4$ |
|---|---|---|---|---|---|
| 2.1 | $2-OCCl=CHCl-C_6H_4-$ | H | O | $CH_3$ | $CH_3$ |

(Siehe Tabelle 3 Seite 16 f.)

Tabelle 3 : Verbindungen der Formel Ic

| Verb. Nr. | $R_1$ | $R_2$ | Z | $R_3$ | $R_4$ | Smp. [°C] |
|---|---|---|---|---|---|---|
| 3.1 | 2-S-CCl=CHCl-$C_6H_4$- | H | O | $CH_3$ | $CH_3$ | |
| 3.2 | 2-S-CCl=CHCl-$C_6H_4$- | H | O | $CH_3$ | $C_2H_5$ | |
| 3.3 | 2-S-CCl=CHCl-$C_6H_4$- | H | O | $CH_3$ | cycl.$C_3H_5$ | |
| 3.4 | 2-S-CCl=CHCl-$C_6H_4$- | H | O | $CH_3$ | $OCH_3$ | |
| 3.5 | 2-S-CCl=CHCl-$C_6H_4$- | H | O | $C_2H_5$ | $CH_3$ | |
| 3.6 | 2-S-CCl=CHCl-$C_6H_4$- | H | O | $C_2H_5$ | $C_2H_5$ | |
| 3.7 | 2-O-$CH_2$-CCl=CHCl-$C_6H_4$- | H | O | $CH_3$ | $CH_3$ | |
| 3.8 | 2-O-$CH_2$-CCl=CHCl-$C_6H_4$- | H | O | $CH_3$ | $C_2H_5$ | |
| 3.9 | 2-O-$CH_2$-CCl=CHCl-$C_6H_4$- | H | O | $CH_3$ | cycl.$C_3H_5$ | |
| 3.10 | 2-S-$CH_2$-CCl=CHCl-$C_6H_4$- | H | O | $CH_3$ | $CH_3$ | |
| 3.11 | 2-S-$CH_2$-CCl=CHCl-$C_6H_4$- | H | O | $CH_3$ | $C_2H_5$ | |
| 3.12 | 2-S-$CH_2$-CCl=CHCl-$C_6H_4$- | H | O | $CH_3$ | cycl.$C_3H_5$ | |

Tabelle 4

| Verb. Nr. | $R_3$ | $R_4$ | Smp. [°C] |
|---|---|---|---|
| 4.1 | $CH_3$ | $OCHF_2$ | 138-140 |
| 4.2 | $C_2H_5$ | $OCHF_2$ | |
| 4.3 | $C_3H_7$-i | $OCHF_2$ | |
| 4.4 | $CH_3$ | $SCHF_2$ | |
| 4.5 | $C_2H_5$ | $SCHF_2$ | |
| 4.6 | $C_3H_7$-i | $SCHF_2$ | |

(Siehe Tabelle 5 Seite 17 f.)

Tabelle 5 : Verbindungen der Formel

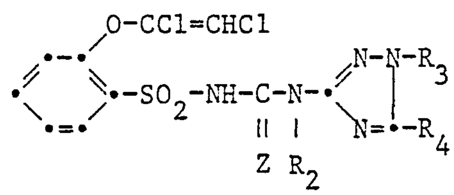

| Verb. Nr. | $R_2$ | Z | $R_3$ | $R_4$ | Smp. [°C] |
|---|---|---|---|---|---|
| 5.1 | H | O | $CH_3$ | $CH_3$ | 202-204 |
| 5.2 | H | S | $CH_3$ | $CH_3$ | |
| 5.3 | $CH_3$ | O | $CH_3$ | $CH_3$ | |
| 5.4 | H | O | $CH_3$ | $C_2H_5$ | 175-185 |
| 5.5 | H | S | $CH_3$ | $C_2H_5$ | |
| 5.6 | $CH_3$ | O | $CH_3$ | $C_2H_5$ | |
| 5.7 | H | O | $CH_3$ | $C_3H_7$-i | 185-190 |
| 5.8 | H | S | $CH_3$ | $C_3H_7$-i | |
| 5.9 | H | O | $CH_3$ | Cycl.$C_3H_5$ | 182-183 |
| 5.10 | H | S | $CH_3$ | Cycl.$C_3H_5$ | |
| 5.11 | $CH_3$ | O | $CH_3$ | Cycl.$C_3H_5$ | |
| 5.12 | H | O | $C_2H_5$ | $CH_3$ | 194-195 |
| 5.13 | H | O | $C_2H_5$ | $C_2H_5$ | 201-203 |
| 5.14 | H | S | $C_2H_5$ | $C_2H_5$ | |
| 5.15 | H | O | $C_2H_5$ | $C_3H_7$-i | |
| 5.16 | H | O | $C_2H_5$ | Cycl.$C_3H_5$ | |
| 5.17 | H | O | $CH_3$ | $OCH_3$ | 182-185 |
| 5.18 | H | O | $CH_3$ | $OC_2H_5$ | |
| 5.19 | H | O | $CH_3$ | $OC_3H_7$-i | |
| 5.20 | H | S | $CH_3$ | $OCH_3$ | |
| 5.21 | $CH_3$ | O | $CH_3$ | $OCH_3$ | |
| 5.22 | H | O | $CH_3$ | $SCH_3$ | 180-184 |
| 5.23 | H | S | $CH_3$ | $SCH_3$ | |
| 5.24 | $CH_3$ | O | $CH_3$ | $SCH_3$ | |
| 5.25 | H | O | $CH_3$ | $N(CH_3)_2$ | 214-215 |
| 5.26 | H | O | $CH_3$ | $NHCH_3$ | |
| 5.27 | H | O | $C_2H_5$ | $N(CH_3)_2$ | |

Tabelle 5 (Fortsetzung)

| Verb.Nr. | $R_2$ | Z | $R_3$ | $R_4$ | Smp.[°C] |
|---|---|---|---|---|---|
| 5.28 | H | O | $C_2H_5$ | $NHCH_3$ | |
| 5.29 | H | S | $CH_3$ | $N(CH_3)_2$ | |
| 5.30 | $CH_3$ | O | $CH_3$ | $N(CH_3)_2$ | |
| 5.31 | H | O | $C_3H_7$-i | $CH_3$ | |
| 5.32 | H | O | $C_3H_7$-i | $C_2H_5$ | |
| 5.33 | H | O | $C_3H_7$-i | $C_3H_7$-i | |
| 5.34 | H | O | $C_3H_7$-i | Cycl.$C_3H_5$ | |
| 5.35 | H | O | $C_3H_7$-i | $OCH_3$ | |
| 5.36 | H | O | $C_3H_7$-i | $SCH_3$ | |
| 5.37 | H | O | $C_3H_7$-i | $N(CH_3)_2$ | |
| 5.38 | H | O | $CH_3$ | $OCHF_2$ | 149-150 |
| 5.39 | H | O | $C_2H_5$ | $OCHF_2$ | |
| 5.40 | H | O | $C_3H_7$-i | $OCHF_2$ | |
| 5.41 | H | O | $CH_3$ | $SCHF_2$ | |
| 5.42 | H | O | $C_2H_5$ | $SCHF_2$ | |
| 5.43 | H | O | $C_3H_7$-i | $SCHF_2$ | |
| 5.44 | H | O | $CH_3$ | Cl | |
| 5.45 | H | O | $C_2H_5$ | Cl | |
| 5.46 | H | O | $C_3H_7$-i | Cl | |

Tabelle 6 : Verbindungen der Formel

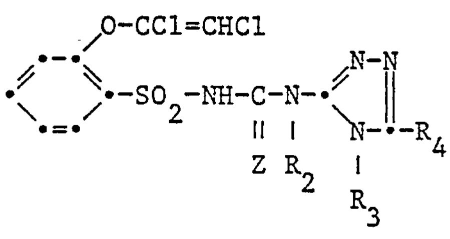

| Verb.Nr. | $R_2$ | Z | $R_3$ | $R_4$ |
|---|---|---|---|---|
| 6.1 | H | O | $CH_3$ | $CH_3$ |
| 6.2 | H | S | $CH_3$ | $CH_3$ |
| 6.3 | $CH_3$ | O | $CH_3$ | $CH_3$ |
| 6.4 | H | O | $CH_3$ | $OCH_3$ |

Tabelle 6 (Fortsetzung)

| Verb.Nr. | $R_2$ | Z | $R_3$ | $R_4$ |
|---|---|---|---|---|
| 6.5 | H | S | $CH_3$ | $OCH_3$ |
| 6.6 | $CH_3$ | O | $CH_3$ | $OCH_3$ |
| 6.7 | H | O | $CH_3$ | $SCH_3$ |
| 6.8 | H | O | $C_2H_5$ | $CH_3$ |
| 6.9 | H | O | $C_2H_5$ | $C_2H_5$ |
| 6.10 | H | O | $C_2H_5$ | $OCH_3$ |
| 6.11 | H | O | $C_2H_5$ | $SCH_3$ |

Tabelle 7 : Verbindungen der Formel

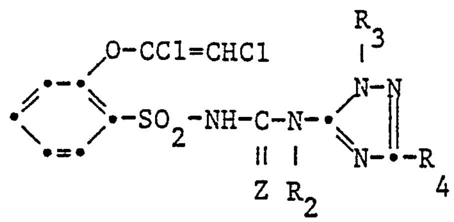

| Verb. Nr. | $R_2$ | Z | $R_3$ | $R_4$ | Smp. [°C] |
|---|---|---|---|---|---|
| 7.1 | H | O | $CH_3$ | $CH_3$ | 161-166 |
| 7.2 | H | O | $CH_3$ | $C_2H_5$ | |
| 7.3 | H | S | $CH_3$ | $CH_3$ | |
| 7.4 | H | O | $C_2H_5$ | $CH_3$ | |
| 7.5 | H | O | $C_3H_7$-i | $CH_3$ | |
| 7.6 | H | O | $C_2H_5$ | $C_2H_5$ | |
| 7.7 | H | O | $C_3H_7$-i | $C_2H_5$ | |
| 7.8 | H | S | $C_3H_7$-i | $C_2H_5$ | |
| 7.9 | H | O | H | $CH_3$ | |
| 7.10 | H | O | H | $C_2H_5$ | |
| 7.11 | H | S | H | $CH_3$ | |
| 7.12 | H | O | $CH_3$ | $C_3H_7$-i | |
| 7.13 | H | O | $C_3H_7$-i | $C_3H_7$-i | |
| 7.14 | H | O | $CH_3$ | $OCH_3$ | 162-166 |

Tabelle 7 (Fortsetzung)

| Verb. Nr. | $R_2$ | Z | $R_3$ | $R_4$ | Smp. [°C] |
|---|---|---|---|---|---|
| 7.15 | H | O | $C_3H_7$-i | $OCH_3$ | 177-179 |
| 7.16 | H | O | $C_4H_9$-t | $OCH_3$ | 156-159 |
| 7.17 | H | S | $CH_3$ | $OCH_3$ | |
| 7.18 | H | O | H | $OCH_3$ | 220 |
| 7.19 | H | O | H | $OC_2H_5$ | 218 |
| 7.20 | H | O | $CH_3$ | $OC_2H_5$ | 157-159 |
| 7.21 | H | O | $C_3H_7$-i | $OC_2H_5$ | 152-154 |
| 7.22 | H | O | $C_4H_9$-t | $OC_2H_5$ | 142-144 |
| 7.23 | H | O | $CH_3$ | H | 176-177 |
| 7.24 | H | O | H | $N(CH_3)_2$ | 195-197 |
| 7.25 | H | O | $CH_3$ | $N(CH_3)_2$ | – |
| 7.26 | H | O | $C_3H_7$-i | $N(CH_3)_2$ | |
| 7.27 | $CH_3$ | O | $CH_3$ | $CH_3$ | |

Zur Applikation können die Verbindungen der Formel I in den folgenden Aufarbeitungsformen vorliegen.

Formulierungsbeispiele

Beispiel 8 : Formulierungsbeispiele für Wirkstoffe der Formel I (% = Gewichtsprozent)

| a) Spritzpulver | a) | b) | c) |
|---|---|---|---|
| Wirkstoff | 20% | 60% | 0,5% |
| Na-Ligninsulfonat | 5% | 5% | 5% |
| Na-Laurylsulfat | 3% | - | - |
| Na-Diisobutylnaphthalinsulfonat | - | 6% | 6% |
| Octylphenolpolyäthylenglykoläther (7-8 Mol AeO) | - | 2% | 2% |
| Hochdisperse Kieselsäure | 5% | 27% | 27% |
| Kaolin | 67% | - | - |
| Natriumchlorid | - | - | 59,5% |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

(Siehe Tabelle Seite 21 f.)

b) Emulsion-Konzentrat

| | a) | b) |
|---|---|---|
| Wirkstoff | 10% | 1% |
| Octylphenolpolyäthylenglykoläther (4-5 Mol AeO) | 3% | 3% |
| Ca-Dodecylbenzolsulfonat | 3% | 3% |
| Ricinusölpolyglykoläther (36 Mol AeO) | 4% | 4% |
| Cyclohexanon | 30% | 10% |
| Xylolgemisch | 50% | 79% |

Aus diesem Konzentrat können durch Verdünnen mit Wasser Emulsionen jeder gewünschten Konzentration hergestellt werden.

c) Stäubemittel

| | a) | b) |
|---|---|---|
| Wirkstoff | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

d) Extruder Granulat

| | a) | b) |
|---|---|---|
| Wirkstoff | 10% | 1% |
| Na-Ligninsulfonat | 2% | 2% |
| Carboxymethylcellulose | 1% | 1% |
| Kaolin | 87% | 96% |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| | |
|---|---|
| Wirkstoff | 3% |
| Polyäthylenglykol (MG 200) | 3% |
| Kaolin | 94% |

Der feingemahlenen Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat

| | a) | b) |
|---|---|---|
| Wirkstoff | 40% | 5% |
| Aethylenglykol | 10% | 10% |
| Nonylphenolpolyäthylenglykoläther (15 Mol AeO) | 6% | 1% |
| Na-Ligninsulfonat | 10% | 5% |
| Carboxymethylcellulose | 1% | 1% |
| 37%ige wässrige Formaldehyd-Lösung | 0,2% | 0,2% |
| Silikonöl in Form einer 75%igen wässrigen Emulsion | 0,8% | 0,8% |
| Wasser | 32% | 77% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) Salzlösung

| | |
|---|---|
| Wirkstoff | 5% |
| Isopropylamin | 1% |
| Octylphenolpolyäthylenglykoläther (78 Mol AeO) | 3% |
| Wasser | 91% |

Aus solchen Konzentration können durch Verdünnen mit Wasser Emulsionen der gewünschten Anwendungskonzentration hergestellt werden, die besonders zur Blattapplikation geeignet sind. Darüberhinaus können weitere Spritzpulver mit anderen Mischungsverhältnissen oder anderen in der Formulierungstechnik gebräuchlichen Trägermaterialien und Zuschlagstoffen hergestellt werden. Die Wirkstoffe werden in geeigneten Mischern mit den genannten Zuschlagstoffen innig vermischt und auf entsprechenden Mühlen und Walzen vermahlen. Man erhält Spritzpulver von vorzüglicher Benetzbarkeit und Schwebefähigkeit, die sich mit Wasser zu Suspensionen der gewünschten Konzentration verdünnt und insbesondere zur Blattapplikation verwenden lassen. Solche Mittel gehören ebenfalls zur Erfindung.

Mittel, die nach der oben beschriebenen Art formuliert wurden und als Wirkungskomponente eine der Verbindungen aus der Tabelle 1 enthielten, liessen sich mit sehr gutem Erfolg pflanzenwuchsregulatorisch pflanzenbakterizid und/oder herbizid einsetzen.

## Biologische Beispiele

### Beispiel 9 : Herbizidwirkung vor dem Auflaufen der Pflanzen

Kunstofftöpfe werden mit expandiertem Vermiculit (Dichte : 0,135 g/cm$^3$, Wasserabsorptionsvermögen : 0,565 1/1) gefüllt. Nach dem Sättigen des nicht adsorptiven Vermiculits mit einer wässrigen Wirkstoffemulsion in deionisiertem Wasser, die die Wirkstoffe in einer Konzentration von 70,8 ppm enthält, werden Samen der folgenden Pflanzen auf die Oberfläche gesät : Nasturtium officinalis, Agrostis tenuis, Stellaria media und Digitaria sanguinalis. Die Versuchsgefässe werden anschliessend in einer Klimakammer bei 20 °C, einer Beleuchtung von ca. 20 kLux und einer relativen Luftfeuchtigkeit von 70 % gehalten. Während der Keimphase von 4 bis 5 Tagen werden die Töpfe zur Erhöhung der örtlichen Luftfeuchtigkeit mit lichtdurchlässigem Material abgedeckt und mit deionisiertem Wasser gegossen. Nach dem 5. Tag wird dem Giesswasser 0,5 % eines handelsüblichen Flüssigdüngers (®Greenzit) zugesetzt. 12 Tage nach der Aussaat wird der Versuch ausgewertet und die Wirkung auf die Versuchspflanzen nach dem folgenden Massstab bewertet :

1 : Pflanze nicht gekeimt oder total abgestorben
2-3 : sehr starke Wirkung
4-6 : mettlere Wirkung
7-8 : schwache Wirkung
9 : keine Wirkung (wie unbehandelte Kontrolle).

pre-emergente Wirkung

Konzentration der Wirkstoffemulsion : 70,8 ppm

| Testpflanze / Wirkstoff Nr. | Nasturtium | Stellaria | Agrostis | Digitaria |
|---|---|---|---|---|
| 1.1 | 2 | 1 | 1 | 2 |
| 1.5 | 1 | 1 | 1 | 2 |
| 5.38 | 2 | 2 | 1 | 2 |

### Beispiel10 : Nachweis der Herbizidwirkung nach dem Auflaufen der Pflanzen (Kontaktwirkung)

Eine grössere Anzahl Unkräuter und Kulturpflanzen, sowohl monokotyle wie dikotyle, wurden nach

dem Auflaufen, im 4- bis 6-Blattstadium mit einer wässrigen Wirkstoffdispersion in verschiedenen Dosierungen auf die Pflanzen gespritzt und diese bei 24° bis 26 °C und 45-60 % relativer Luftfeuchtigkeit gehalten. 15 Tage nach der Behandlung zeigten die Verbindungen der Formel I gute Herbizidwirkung.

Beispiel 11 : Wuchshemmung bei Getreide

In Kunststofftöpfen mit sterilisierter Erde wurden die Getreidearten Hordeum vulgare (Sommergerste) und Secale (Sommerroggen) im Gewächshaus angesät und nach Bedarf bewässert. Die Sprösslinge wurden ca. 21 Tage nach der Aussaat mit der wässrigen Spritzbrühe eines Wirkstoffes der Formel I besprüht. Die Wirkstoffmenge betrug umgerechnet 0,5 bzw. 2,5 kg Aktivsubstanz per Hektar. 10 und 21 Tage nach Applikation wurde das Wachstum des Getreides beurteilt. Hierbei konnte festgestellt werden, dass Getreidepflanzen die mit Wirkstoffen der Formel I behandelt worden waren, im Vergleich zu unbehandelten Kontrollpflanzen eine starke Wuchsreduktion aufwiesen.

Beispiel 12 : Wuchshemmung bei Gräsern

In Kunststoffschalen mit Erde-Topf-Sand-Gemisch (6 : 3 : 1) wurden die Gräser Lolium perenne, Poa pratensis, Festuca ovina und Cynodon dactylon im Gewächshaus angesät und nach Bedarf bewässert. Die aufgelaufenen Gräser wurden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge betrug umgerechnet 2,5 kg Aktivsubstanz per Hektar. 10 und 21 Tage nach Applikation wurde das Wachstum der Gräsern beurteilt, dabei zeigte es sich, dass die erfindungsgemässen Wirkstoffe aus der Tabelle 1 eine merkliche Wuchshemmung bewirkten.

Beispiel 13 : Wirkung gegen Xanthomonas oryzae auf Reis

a) Residual-protektive Wirkung

Reispflanzen der Sorte « Caloro » oder « S6 » wurden nach 3-wöchiger Anzucht im Gewächshaus mit der Prüfsubstanz in Form einer Spritzbrühe (Konzentrationen 0,2 %, 0,06 %, 0,02 % Aktivsubstanz) besprüht. Nach eintägigem Antrocknen dieses Spritzbelages wurden die Pflanzen in einem Klimaraum bei 24 °C und 75-85 % relativer Luftfeuchtigkeit aufgestellt und infiziert. Die Infektion erfolgte, indem die Blattspitzen mit einer Schere, die zuvor in eine Suspension von Xanthomonas oryzae eingetaucht worden war, abgeschnitten wurden. Nach 10-tägiger Inkubation in demselben Raum wurden die angeschnittenen Blätter welk, rollten sich ein und wurden nekrotisch. Das Ausmass dieser Krankheitssymptome diente zur Beurteilung der residualen Wirksamkeit der Prüfsubstanz.

In den obigen Versuchen zeigten die Verbindungen der Formel IX eine gute Wirkung.

Die Verbindungen 5.1, 5.4, 5.7, 5.9, 5.17 und 5.25 verhinderten bei einer Konzentration von 0,02 % den Befall zu 95-100 %, die Verbindung 5.22 zu 90-95 %.

b) Systemische Wirkung

Reispflanzen der Sorte « Caloro » oder « S6 » wurden nach 3-wöchiger Anzucht im Gewächshaus mit einer Suspension der Prüfsubstanz begossen (Konzentrationen : 0,06 %, 0,02 % und 0,006 % Aktivsubstanz bezogen auf das Erdvolumen). Drei Tage nach dieser Behandlung wurden die Pflanzen in einem Klimaraum bei 24 °C und 75-85 % relativer Luftfeuchtigkeit aufgestellt und infiziert. Die Infektion erfolgte, indem die Blattspitzen mit einer Schere, die zuvor in eine Suspension von Xanthomonas oryzae eingetaucht worden war, abgeschnitten wurden. Nach 10-tägiger Inkubation in demselben Raum wurden die angeschnittenen Blätter welk, rollten sich ein und wurden nekrotisch. Das Ausmass dieser Krankheitssymptome diente zur Beurteilung der systemischen Wirksamkeit der Prüfsubstanz.

In den obigen Versuchen zeigten die Verbindungen der Formel IX eine gute Wirkung. Die Verbindungen 5.1, 5.9, 5.22 und 5.25 verhinderten bei einer Konzentration von 0,006 % den Befall zu 90-100 %, die Verbindung 5.7 bei einer Konzentration von 0,02 % zu 95-100 %.

Beispiel 14 : Wirkung gegen Xanthomonas vesicatoria auf Paprika

a) Residual-protektive Wirkung

Paprikapflanzen der Sorte « California Wonder » wurden nach 3-wöchiger Anzucht im Gewächshaus mit der Prüfsubstanz in Form einer Spritzbrühe (0,06 % Aktivsubstanz) besprüht. Nach eintägigem Antrocknen dieses Spritzbelages wurden die Pflanzen in einem Klimaraum bei 26 °C und 95-100 % relativer Luftfeuchtigkeit aufgestellt und durch Besprühen der Blattunterseiten mit einer standardisierten Suspension von Xanthomonas vesicatoria infiziert. Nach 6-tägiger Inkubation in demselben Raum entstanden auf den Blättern runde, anfangs wässrige, später nekrotische, aufgehellte Flecken. Das Ausmass dieser Flecken diente zur Beurteilung der residualen Wirksamkeit der Prüfsubstanz.

b) Systemische Wirkung

Paprikapflanzen der Sorte « California Wonder » wurden nach 3-wöchiger Anzucht im Gewächshaus mit einer Suspension der Prüfsubstanz begossen (0,006 % Aktivsubstanz bezogen auf das Erdvolumen). Drei Tage nach dieser Behandlung wurden die Pflanzen in einem Klimaraum bei 26 °C und 95-100 % realtiver Luftfeuchtigkeit aufgestellt und durch Besprühen der Blattunterseiten mit einer standardisierten Suspension von Xanthomonas vesicatoria infiziert. Nach 6-tägiger Inkubation in demselben Raum entstanden auf den Blättern runde, anfangs wässrige, später nekrotische, aufgehellte Flecken. Das Ausmass dieser Flecken diente zur Beurteilung der systemischen Wirksamkeit der Prüfsubstanz.

In den obigen Versuchen 14a und 14b zeigten die Verbindungen der Formel IX eine gute Wirkung.

**Patentansprüche** (für die Vertragsstaaten : BE, CH, DE, FR, GB, IT, LI, NL, SE)

1. N-Arylsulfonyl-N'-triazolyl-harnstoff der Formel I

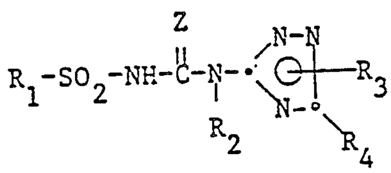

(I)

worin

$R_1$ einen Rest der Formel

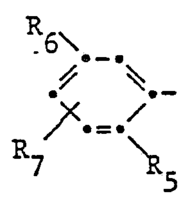

oder

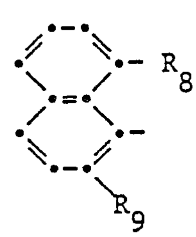

$R_2$ und $R_3$ unabhängig voneinander Wasserstoff oder $C_1$-$C_6$-Alkyl,

$R_4$ Wasserstoff, Halogen, $C_1$-$C_6$-Alkyl, $C_1$-$C_6$-Halogenalkyl, Cyclopropyl, $-NR_{10}R_{11}$ oder $-X-R_{12}$ und

Z Sauerstoff oder Schwefel bedeuten,

wobei

$R_5$ und $R_9$ Wasserstoff, Halogen, Cyan, Nitro, $C_1$-$C_6$-Alkyl, $C_1$-$C_6$-Halogen-alkyl, gegebenenfalls durch 1 bis 3 Halogenatome substituiertes $C_2$-$C_6$-Alkenyl, $-CO-R_{13}$, $-NR_{10}R_{11}$ $-SO_2-C_1$-$C_4$-Halogenalkoxy, $-SO_2-NR_{10}R_{11}$, $-NH-SO_2-R_{15}$, $-O-SO_2-R_{15}$, $-N(C_1$-$C_4$-Alkyl)$-SO_2-R_{15}$, oder einen gegebenenfalls durch einen oder mehrere Substituenten aus der Reihe Halogen, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Halogenalkoxy, $C_1$-$C_4$-Halogenalkylthio, $C_1$-$C_4$-Halogenalkylsulfinyl oder $C_1$-$C_4$-Halogensulfonyl substituierten Rest $-Y-R_{14}$,

$R_6$ Wasserstoff, Halogen, Nitro oder $C_1$-$C_6$-Alkoxy,

$R_7$ und $R_8$ Wasserstoff, Halogen, Nitro, $C_1$-$C_6$-Halogenalkyl, $-NH_2$, $C_1$-$C_6$-Alkyl, $C_1$-$C_6$-Alkoxy, $C_1$-$C_6$-Alkylthio, $C_1$-$C_6$-Alkylsulfinyl oder $C_1$-$C_6$-Alkylsulfonyl,

$R_{10}$ und $R_{11}$ unabhängig voneinander Wasserstoff, $C_1$-$C_4$-Alkyl oder Cyclopropyl oder $R_{10}$ und $R_{11}$ zusammen eine gegebenenfalls durch Methyl substituierte oder Sauerstoff, Schwefel, $-SO-$, $-SO_2-$, $-NH-$ oder $-N(C_1$-$C_4$-Alkyl)$-$ unterbrochene $C_4$-$C_6$-Alkylenbrücke,

$R_{12}$ $C_1$-$C_4$-Alkyl, $C_1$-$C_4$-Halogenalkyl oder Alkoxyalkyl mit höchstens 4 Kohlenstoffatomen,

$R_{13}$ Wasserstoff, $C_1$-$C_4$-Alkyl, $C_3$-$C_6$-Alkenyloxy, $C_3$-$C_6$-Alkinyloxy, $C_1$-$C_4$-Halogenalkyl, $C_1$-$C_6$-Alkylthio, Phenoxy, Benzyloxy, $-NR_{10}R_{11}$ oder gegebenenfalls durch 1 bis 3 Halogenatome substituiertes $C_1$-$C_6$-Alkoxy,

$R_{14}$ $C_1$-$C_6$-Alkyl, $C_2$-$C_6$-Alkenyl oder $C_3$-$C_6$-Alkinyl

$R_{15}$ $C_1$-$C_6$-Alkyl oder $C_1$-$C_6$-Halogenalkyl und

X und Y unabhängig voneinander Sauerstoff, Schwefel, eine Sulfinyl- oder Sulfonylbrücke bedeuten, sowie Salze dieser Verbindungen ;

mit der Massgabe, dass

a) $R_1$ nur dann die angegebene Bedeutung hat, wenn $R_4$ für die Gruppe —X—$CHF_2$ steht, und dass

b) $R_4$ nur dann die angegebene Bedeutung hat, wenn $R_1$ für den substituierten Phenylkern und $R_5$ für die Gruppe —Y—$C_2$-$C_6$-Halogenalkyl stehen.

2. Verbindungen gemäss Anspruch 1, dadurch gekennzeichnet, dass

$R_1$ einen Rest der Formel

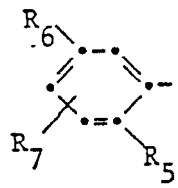

oder

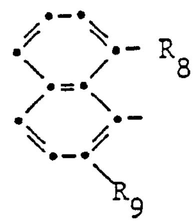

$R_2$ und $R_3$ unabhängig voneinander Wasserstoff oder $C_1$-$C_4$-Alkyl,

$R_4$ Wasserstoff, Halogen, $C_1$-$C_4$-Alkyl, $C_1$-$C_4$-Halogenalkyl, Cyclopropyl, —$NR_{10}R_{11}$ oder —X—$R_{12}$ und

Z Sauerstoff oder Schwefel bedeuten,

wobei

$R_5$ Wasserstoff, Halogen, Cyan, Nitro, $C_1$-$C_4$-Alkyl, $C_1$-$C_4$-Halogenalkyl, $C_2$-$C_6$-Alkenyl, —CO—$R_{13}$, —$NR_{10}R_{11}$ oder einen gegebenenfalls durch einen oder mehrere Substituenten aus der Reihe Halogen, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Halogenalkoxy, $C_1$-$C_4$-Halogenalkylthio, $C_1$-$C_4$-Halogenalkylsulfinyl oder $C_1$-$C_4$-Halogensulfonyl substituierten Rest —Y—$R_{14}$,

$R_6$ Wasserstoff, Halogen, Nitro oder $C_1$-$C_4$-Alkoxy,

$R_7$ Wasserstoff, Halogen, Nitro, $C_1$-$C_4$-Halogenalkyl, —$NH_2$, $C_1$-$C_4$-Alkyl, $C_1$-$C_4$-Alkoxy, $C_1$-$C_3$-Alkylthio, $C_1$-$C_3$-Alkylsulfonyl oder $C_1$-$C_3$-Alkylsulfonyl,

$R_{10}$ und $R_{11}$ unabhängig voneinander Wasserstoff, $C_1$-$C_3$-Alkyl oder Cyclopropyl oder $R_{10}$ und $R_{11}$ zusammen eine gegebenenfalls durch Methyl substituierte oder Sauerstoff, Schwefel, —SO—, —$SO_2$—, —NH— oder —N($C_1$-$C_4$-Alkyl) unterbrochene $C_4$-$C_6$-Alkylenbrücke,

$R_{12}$ $C_1$-$C_4$-Alkyl, $C_1$-$C_4$-Halogenalkyl oder Alkoxyalkyl mit höchstens 4 Kohlenstoffatomen,

$R_{13}$ Wasserstoff, $C_1$-$C_4$-Alkyl, $C_3$-$C_5$-Alkenyloxy, $C_3$-$C_5$-Alkinyloxy, $C_1$-$C_5$-Alkylthio, Phenoxy, Benzyloxy, —$NR_{10}R_{11}$ oder gegebenenfalls durch 1 bis 3 Halogenatome substituiertes $C_1$-$C_5$-Alkoxy,

$R_{14}$ $C_1$-$C_6$-Alkyl, $C_2$-$C_6$-Alkenyl oder $C_3$-$C_6$-Alkinyl und

X und Y unabhängig voneinander Sauerstoff, Schwefel, eine Sulfinyl- oder Sulfonylbrücke bedeuten, wobei $R_8$ die gleiche Bedeutung wie $R_7$ und $R_9$ die gleiche Bedeutung wie $R_5$ haben, sowie Salze dieser Verbindungen ; mit der Massgabe, dass

a) $R_1$ nur dann die angegebene Bedeutung hat, wenn $R_4$ für die Gruppe —X—$CHF_2$ steht, und dass

b) $R_4$ nur dann die angegebene Bedeutung hat, wenn $R_1$ für den substituierten Phenylkern und $R_5$ für die Gruppe —Y—$C_2$-$C_6$-Halogenalkyl stehen.

3. Verbindungen der Formel I nach Anspruch 1, dadurch gekennzeichnet, dass der Substituent $R_1$ für die Gruppe

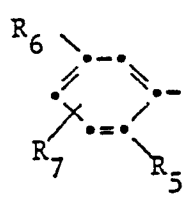

steht, wobei $R_5$ für die Gruppe —Y—$C_2$-$C_6$-Halogenalkyl steht und die Substituenten $R_6$ und $R_7$ die unter der Formel I angegebenen Bedeutungen haben.

4. Verbindungen gemäss Anspruch 1, dadurch gekennzeichnet, dass $R_4$ für die Gruppe —X—$CHF_2$ steht.

5. Verbindungen gemäss Anspruch 3, dadurch gekennzeichnet, dass sie der Formel IX

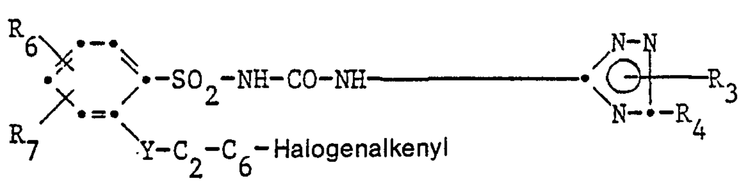

(IX)

entsprechen, worin $R_3$ für Wasserstoff oder $C_1$-$C_4$-Alkyl, $R_4$ für $C_1$-$C_4$-Alkoxy, $C_1$-$C_4$-Alkylthio, —N—di-$C_1$-$C_4$-Alkyl, —$SO_2$—$C_1$-$C_4$-Alkyl oder $C_1$-$C_4$-Halogenalkoxy und Y für Sauerstoff oder Schwefel stehen und $R_6$ und $R_7$ die unter Formel I in Anspruch gegebene Bedeutung haben.

6. Verbindungen gemäss Anspruch 5, dadurch gekennzeichnet, dass $R_6$ und $R_7$ Wasserstoff bedeuten.

7. Verbindungen gemäss Anspruch 6, dadurch gekennzeichnet, dass die —Y—$C_2$-$C_6$-Halogenalkenylgruppe für 1,2-Dichlorvinyloxy oder 1,2-Dichlorvinylthio bedeutet.

8. Verbindungen gemäss Anspruch 7, dadurch gekennzeichnet, dass die —Y—$C_2$-$C_6$-Halogenalkenylgruppe für 1,2-Dichlorvinyloxy steht.

9. Verbindungen gemäss Anspruch 1, dadurch gekennzeichnet, dass $R_1$ für in 2-Stellung durch Fluor, Chlor, Nitro, $C_1$-$C_4$-Alkoxycarbonyl, $C_2$-$C_5$-Alkoxyalkoxy, $C_1$-$C_4$-Halogenalkoxy, $C_1$-$C_4$-Halogenalkylthio, $C_1$-$C_4$-Halogenalkoxy oder —Y—$C_2$-$C_6$-Halogenalkenyl substituiertes Phenyl, $R_2$ für Wasserstoff, Z für Sauerstoff, $R_3$ für Wasserstoff oder $C_1$-$C_4$-Alkyl, $R_4$ für —X—$CHF_2$ und X und Y unabhängig voneinander für Sauerstoff oder Schwefel stehen.

10. Verbindungen gemäss Anspruch 1, dadurch gekennzeichnet, dass sie der Formel Ia

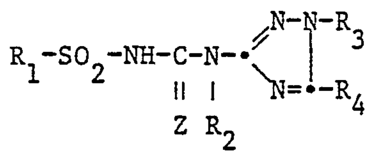

(Ia)

entsprechen, worin $R_1$ bis $R_4$ und Z die unter Formel I in Anspruch 1 gegebene Bedeutung haben.

11. Verbindungen gemäss Anspruch 10, dadurch gekennzeichnet, dass $R_1$ für in 2-Stellung durch eine Gruppe —Y—$C_2$-$C_6$-Halogenalkenyl substituiertes Phenyl, $R_2$ für Wasserstoff, Z für Sauerstoff, $R_3$ für Wasserstoff oder $C_1$-$C_4$-Alkyl, $R_4$ für $C_1$-$C_4$-Alkoxy, $C_1$-$C_4$-Alkylthio, —N—di-$C_1$-$C_4$-Alkyl, —$SO_2$—$C_1$-$C_4$-Alkyl oder $C_1$-$C_4$-Halogenalkoxy und Y für Sauerstoff oder Schwefel stehen.

12. Verbindungen gemäss Anspruch 11, dadurch gekennzeichnet, dass $R_1$ für 2-(1,2-Dichlorvinyloxy)-phenyl] oder 2-(1,2-Dichlorvinylthio)-phenyl steht.

13. Verbindungen gemäss Anspruch 12, dadurch gekennzeichnet, dass $R_1$ für 2-(1,2-Dichlorvinyloxy)-phenyl steht.

14. Verbindungen gemäss Anspruch 10, dadurch gekennzeichnet, dass $R_1$ für in 2-Stellung durch Fluor, Chlor, Nitro, $C_1$-$C_4$-Alkoxycarbonyl, $C_2$-$C_5$-Alkoxyalkoxy, $C_1$-$C_4$-Halogenalkoxy, $C_1$-$C_4$-Halogenalkylthio, $C_1$-$C_4$-Halogenalkyl oder —Y—$C_2$-$C_6$-Halogenalkenyl substituiertes Phenyl, $R_2$ für Wasserstoff, Z für Sauerstoff, $R_3$ für Wasserstoff oder $C_1$-$C_4$-Alkyl, $R_4$ für —X—$CHF_2$ und X und Y unabhängig voneinander für Sauerstoff oder Schwefel stehen.

15. Verbindungen gemäss Anspruch 14, dadurch gekennzeichnet, dass X Sauerstoff bedeutet.

16. Eine Verbindung der Formel I nach Anspruch 1, ausgewählt aus der Reihe :

N-(2-Methoxycarbonylphenyl-sulfonyl)-N'-(1-methyl-5-difluormethoxy-1,2,4-triazol-3-yl)-harnstoff.
N-[2-(1,2-Dichlorvinyloxy)-phenyl-sulfonyl]-N'-(1,5-dimethyl-1,2,4-triazol-3-yl)-harnstoff,
N-[2-(1,2-Dichlorvinyloxy)-phenyl-sulfonyl]-N'-(5-äthyl-1-methyl-1,2,4-triazol-3-yl)-harnstoff,
N-[2-(1,2-Dichlorvinyloxy)-phenyl-sulfonyl]-N'-(1-methyl-5-isopropyl-1,2,4-triazol-3-yl)-harnstoff,
N-[2-(1,2-Dichlorvinyloxy)-phenyl-sulfonyl]-N'-(1-methyl-5-cyclopropyl-1,2,4-triazol-3-yl)-harnstoff,
N-[2-(1,2-Dichlorvinyloxy)-phenyl-sulfonyl]-N'-(1-methyl-5-methylthio-1,2,4-triazol-3-yl)-harnstoff,
N-[2-(1,2-Dichlorvinyloxy)-phenyl-sulfonyl]-N'-(5-dimethylamino-1-methyl-1,2,4-triazol-3-yl)-harnstoff,
N-[2-(1,2-Dichlorvinyloxy)-phenyl-sulfonyl]-N'-(1-äthyl-5-methyl-1,2,4-triazol-3-yl)-harnstoff,
N-[2-(1,2-Dichlorvinyloxy)-phenyl-sulfonyl]-N'-(1,5-diäthyl-1,2,4-triazol-3-yl)-harnstoff,
N-[2-(1,2-Dichlorvinyloxy)-phenyl-sulfonyl]-N'-(5-methoxy-1-methyl-1,2,4-triazol-3-yl)-harnstoff,
N-[2-(1,2-Dichlorvinyloxy)-phenyl-sulfonyl]-N'-(1-methyl-5-difluormethoxy-1,2,4-triazol-3-yl)-harnstoff,
N-[2-(1,2-Dichlorvinyloxy)-phenyl-sulfonyl]-N'-(1,3-dimethyl-1,2,4-triazol-5-yl)-harnstoff.

17. Verfahren zur Herstellung von Verbindungen der Formel I, dadurch gekennzeichnet, dass man ein Phenylsulfonylderivat der Formel II

$$R_1—SO_2W \quad (II)$$

mit einer Verbindung der Formel III

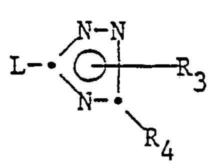

(III)

umsetzt, wobei L und W für eine der Gruppen $—NH_2—$ —N=C=Z oder $—NR_2—CZ—OR$ stehen ; die Substituenten $R_1$, $R_2$, $R_3$, $R_4$ und Z die unter Formel I angegebenen Bedeutungen haben, R für einen aliphatischen oder aromatischen Rest steht ; mit der Massgabe, dass die Reaktionspartner der Formeln II und III stets so gewählt werden, dass eine Aminofunktion entweder mit Isocyanato-, Isothiocyanato- oder mit einer $[—NR_2—CZ—OR]$-Gruppe zur Reaktion gelangt.

18. Verfahren nach Anspruch 17, dadurch gekennzeichnet, dass R für $C_1$-$C_4$-Alkyl, Phenyl oder Benzyl steht.

19. Herbizides, bakterizides und/oder pflanzenwuchsregulierendes Mittel, dadurch gekennzeichnet, dass es als mindestens eine aktive Komponente eine Verbindung der Formel I nach Anspruch 1 enthält.

20. Mittel nach Anspruch 19, dadurch gekennzeichnet, dass es 0,1 bis 95 Gewichtsprozent einer Verbindung der Formel I nach Anspruch 1 und 1 bis 99,9 Gewichtsprozent an Zuschlagstoffen, darunter 0,1 bis 25 Gewichtsprozent eines Tensides, enthält.

21. Verwendung einer Verbindung der Formel I nach Anspruch 1 zur Bekämpfung von unerwünschten Pflanzensorten in Kulturen von Nutzpflanzen und/oder zur Regulierung des Pflanzenwachstums von Nutzpflanzen.

22. Verwendung nach Anspruch 21 zur selektiven Unkrautbekämpfung.

23. Verfahren zur pre- oder post-emergenten Bekämpfung von Unkräutern und/oder zur Beeinflussung des Wachstums von Nutzpflanzen, dadurch gekennzeichnet, dass man eine wirksame Menge eines Wirkstoffes der Formel I gemäss Anspruch 1 auf die Schad- oder Nutzpflanzen, deren Pflanzenteile oder deren Standort appliziert.

24. Verwendung einer Verbindung der Formel IX nach Anspruch 5 zur Bekämpfung phytopathogenen Bakterien.

25. Verwendung gemäss Anspruch 24, dadurch gekennzeichnet, dass die Bakterien zur Familie der Xanthomonoceae oder Pseudomonaceae gehören.

26. Verfahren zur Bekämpfung phytopathogener Bakterien, dadurch gekennzeichnet, dass man eine wirksame Menge eines Wirkstoffes der Formel IX auf die zu schützenden Nutzpflanzen, deren Pflanzenteile oder deren Standort appliziert.

**Patentansprüche** (für den Vertragsstaat AT)

1. Herbizides und/oder pflanzenwuchsregulierendes Mittel, dadurch gekennzeichnet, dass es als mindestens eine aktive Komponente einen N-Arylsulfonyl-N'-triazolyl-harnstoff der Formel I

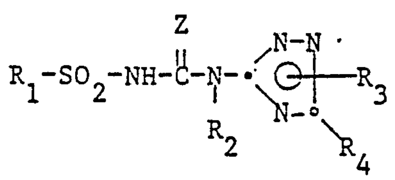

(I)

oder ein Salz davon enthält, worin

$R_1$ einen Rest der Formel

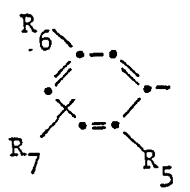

oder

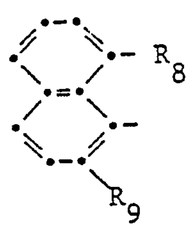

$R_2$ und $R_3$ unabhängig voneinander Wasserstoff oder $C_1$-$C_6$-Alkyl,

$R_4$ Wasserstoff, Halogen, $C_1$-$C_6$-Alkyl, $C_1$-$C_6$-Halogenalkyl, Cyclopropyl, $—NR_{10}R_{11}$ oder $—X—R_{12}$ und

Z Sauerstoff oder Schwefel bedeuten,

wobei

$R_5$ und $R_9$ Wasserstoff, Halogen, Cyan, Nitro, $C_1$-$C_6$-Alkyl, $C_1$-$C_6$-Halogenalkyl, gegebenenfalls durch 1 bis 3 Halogenatome substituiertes $C_2$-$C_6$-Alkenyl, $—CO—R_{13}$, $—NR_{10}R_{11}$ $—SO_2—C_1$-$C_4$-Halogenalkoxy, $—SO_2—NR_{10}R_{11}$, $—NH—SO_2—R_{15}$, $—O—SO_2—R_{15}$, $—N(C_1$-$C_4$-Alkyl$)—SO_2—R_{15}$, oder einen gegebenenfalls durch einen oder mehrere Substituenten aus der Reihe Halogen, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Halogenalkoxy, $C_1$-$C_4$-Halogenalkylthio, $C_1$-$C_4$-Halogenalkylsulfinyl oder $C_1$-$C_4$-Halogensulfonyl substituierten Rest $—Y—R_{14}$,

$R_6$ Wasserstoff, Halogen, Nitro oder $C_1$-$C_6$-Alkoxy,

$R_7$ und $R_8$ Wasserstoff, Halogen, Nitro, $C_1$-$C_6$-Halogenalkyl, $—NH_2$, $C_1$-$C_6$-Alkyl, $C_1$-$C_6$-Alkoxy, $C_1$-$C_6$-Alkylthio, $C_1$-$C_6$-Alkylsulfinyl oder $C_1$-$C_6$-Alkylsulfonyl,

$R_{10}$ und $R_{11}$ unabhängig voneinander Wasserstoff, $C_1$-$C_4$-Alkyl, Cyclopropyl, $C_2$-$C_6$-Alkenyl oder $C_3$-$C_6$-Alkinyl oder $R_{10}$ und $R_{11}$ zusammen eine gegebenenfalls durch Methyl substituierte oder Sauerstoff, Schwefel, —SO—, $—SO_2—$, —NH— oder —N($C_1$-$C_4$-Alkyl)- unterbrochene $C_4$-$C_6$-Alkylenbrücke,

$R_{12}$ $C_1$-$C_4$-Alkyl, $C_1$-$C_4$-Halogenalkyl oder Alkoxyalkyl mit höchstens 4 Kohlenstoffatomen,

$R_{13}$ Wasserstoff, $C_1$-$C_4$-Alkyl, $C_3$-$C_6$-Alkenyloxy, $C_3$-$C_6$-Alkinyloxy, $C_1$-$C_4$-Halogenalkyl, $C_1$-$C_6$-Alkylthio, Phenoxy, Benzyloxy, $—NR_{10}R_{11}$ oder gegebenenfalls durch 1 bis 3 Halogenatome substituiertes $C_1$-$C_6$-Alkoxy,

$R_{14}$ $C_1$-$C_6$-Alkyl, $C_2$-$C_6$-Alkenyl oder $C_3$-$C_6$-Alkinyl

$R_{15}$ $C_1$-$C_6$-Alkyl oder $C_1$-$C_6$-Halogenalkyl und

X und Y unabhängig voneinander Sauerstoff, Schwefel, eine Sulfinyl- oder Sulfonylbrücke bedeuten ; mit der Massgabe, dass

a) $R_1$ nur dann die angegebene Bedeutung hat, wenn $R_4$ für die Gruppe $—X—CHF_2$ steht, und dass

b) $R_4$ nur dann die angegebene Bedeutung hat, wenn $R_1$ für den substituierten Phenylkern und $R_5$ für die Gruppe —Y—$C_2$-$C_6$-Halogenalkyl stehen.

2. Mittel nach Anspruch 1, dadurch gekennzeichnet, dass es 0,1 bis 95 Gewichtsprozent einer Verbindung der Formel I nach Anspruch 1 und 1 bis 99,9 Gewichtsprozent an Zuschlagstoffen, darunter 0,1 bis 25 Gewichtsprozent eines Tensides, enthält.

3. Mittel gemäss Anspruch 1, dadurch gekennzeichnet, dass

$R_1$

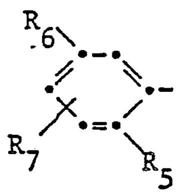

oder

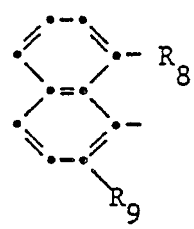

$R_2$ und $R_3$ unabhängig voneinander Wasserstoff oder $C_1$-$C_4$-Alkyl,

$R_4$ Wasserstoff, Halogen, $C_1$-$C_4$-Alkyl, $C_1$-$C_4$-Halogenalkyl, Cyclopropyl, $—NR_{10}R_{11}$ oder $—X—R_{12}$ und

Z Sauerstoff oder Schwefel bedeuten,

wobei

$R_5$ Wasserstoff, Halogen, Cyan, Nitro, $C_1$-$C_4$-Alkyl, $C_1$-$C_4$-Halogenalkyl, $C_2$-$C_6$-Alkenyl, $—CO—R_{13}$, $—NR_{10}R_{11}$ oder einen gegebenenfalls durch einen oder mehrere Substituenten aus der Reihe Halogen, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Halogenalkoxy, $C_1$-$C_4$-Halogenalkylthio, $C_1$-$C_4$-Halogenalkylsulfinyl oder $C_1$-$C_4$-Halogensulfonyl substituierten Rest $—Y—R_{14}$,

$R_6$ Wasserstoff, Halogen, Nitro oder $C_1$-$C_4$-Alkoxy,

$R_7$ Wasserstoff, Halogen, Nitro, $C_1$-$C_4$-Halogenalkyl, $—NH_2$, $C_1$-$C_4$-Alkyl, $C_1$-$C_4$-Alkoxy, $C_1$-$C_3$-Alkylthio, $C_1$-$C_3$-Alkylsulfonyl oder $C_1$-$C_3$-Alkylsulfonyl,

$R_{10}$ und $R_{11}$ unabhängig voneinander Wasserstoff, $C_1$-$C_3$-Alkyl, Cyclopropyl, $C_2$-$C_6$-Alkenyl oder $C_3$-$C_6$-Alkinyl oder $R_{10}$ und $R_{11}$ zusammen eine gegebenenfalls durch Methyl substituierte oder Sauerstoff, Schwefel, —SO—, $—SO_2—$, —NH— oder —N($C_1$-$C_4$-Alkyl) unterbrochene $C_4$-$C_6$-Alkylenbrücke,

$R_{12}$ $C_1$-$C_4$-Alkyl, $C_1$-$C_4$-Halogenalkyl oder Alkoxyalkyl mit höchstens 4 Kohlenstoffatomen,

$R_{13}$ Wasserstoff, $C_1$-$C_4$-Alkyl, $C_3$-$C_5$-Alkenyloxy, $C_3$-$C_5$-Alkinyloxy, $C_1$-$C_5$-Alkylthio, Phenoxy, Benzyloxy, $—NR_{10}R_{11}$ oder gegebenenfalls durch 1 bis 3 Halogenatome substituiertes $C_1$-$C_5$-Alkoxy,

$R_{14}$ $C_1$-$C_6$-Alkyl, $C_2$-$C_6$-Alkenyl oder $C_3$-$C_6$-Alkinyl und

X und Y unabhängig voneinander Sauerstoff, Schwefel, eine Sulfinyl- oder Sulfonylbrücke bedeuten, wobei $R_8$ die gleiche Bedeutung wie $R_7$ und $R_9$ die gleiche Bedeutung wie $R_5$ haben ; mit der Massgabe, dass

a) $R_1$ nur dann die angegebene Bedeutung hat, wenn $R_4$ für die Gruppe $—X—CHF_2$ steht, und dass

b) $R_4$ nur dann die angegebene Bedeutung hat, wenn $R_1$ für den substituierten Phenylkern und $R_5$ für die Gruppe —Y—$C_2$-$C_6$-Halogenalkyl stehen.

4. Mittel nach Anspruch 1, dadurch gekennzeichnet, dass der Substituent $R_1$ für die Gruppe

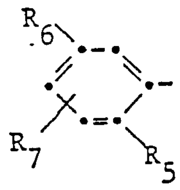

steht, wobei $R_5$ für die Gruppe —Y—$C_2$-$C_6$-Halogenalkenyl steht und die Substituenten $R_6$ und $R_7$ die unter der Formel I angegebenen Bedeutungen haben.

5. Mittel gemäss Anspruch 1, dadurch gekennzeichnet, dass $R_4$ für die Gruppe —X—$CHF_2$ steht.

6. Mittel gemäss Anspruch 4, dadurch gekennzeichnet, dass sie der Formel IX

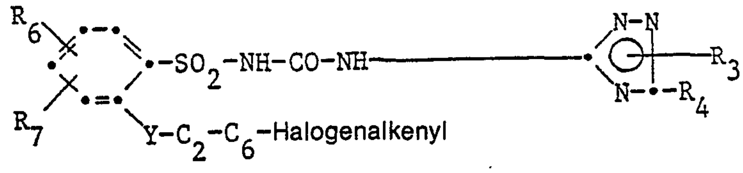

(IX)

entsprechen, worin $R_3$ für Wasserstoff oder $C_1$-$C_4$-Alkyl, $R_4$ für $C_1$-$C_4$-Alkoxy, $C_1$-$C_4$-Alkylthio, —N—di-$C_1$-$C_4$-Alkyl, —$SO_2$-$C_1$-$C_4$-Alkyl oder $C_1$-$C_4$-Halogenalkoxy und Y für Sauerstoff oder Schwefel stehen und $R_6$ und $R_7$ die unter Formel I in Anspruch gegebene Bedeutung haben.

7. Mittel gemäss Anspruch 6, dadurch gekennzeichnet, dass $R_6$ und $R_7$ Wasserstoff bedeuten.

8. Mittel gemäss Anspruch 7, dadurch gekennzeichnet, dass die —Y—$C_2$-$C_6$-Halogenalkenylgruppe für 1,2-Dichlorvinyloxy oder 1,2-Dichlorvinylthio bedeutet.

9. Mittel gemäss Anspruch 8, dadurch gekennzeichnet, dass die —Y—$C_2$-$C_6$-Halogenalkenylgruppe für 1,2-Dichlorvinyloxy steht.

10. Mittel gemäss Anspruch 1, dadurch gekennzeichnet, dass $R_1$ für in 2-Stellung durch Fluor, Chlor, Nitro, $C_1$-$C_4$-Alkoxycarbonyl, $C_2$-$C_5$-Alkoxyalkoxy, $C_1$-$C_4$-Halogenalkoxy, $C_1$-$C_4$-Halogenalkylthio, $C_1$-$C_4$-Halogenalkoxy oder —Y—$C_2$-$C_6$-Halogenalkenyl substituiertes Phenyl, $R_2$ für Wasserstoff, Z für Sauerstoff, $R_3$ für Wasserstoff oder $C_1$-$C_4$-Alkyl, $R_4$ für —X—$CHF_2$ und X und Y unabhängig voneinander für Sauerstoff oder Schwefel stehen.

11. Mittel gemäss Anspruch 1, dadurch gekennzeichnet, dass sie der Formel Ia

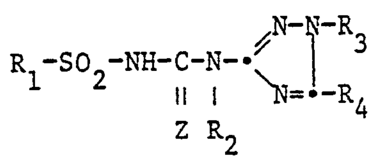

(Ia)

entsprechen, worin $R_1$ bis $R_4$ und Z die unter Formel I in Anspruch 1 gegebenen Bedeutung haben.

12. Mittel gemäss Anspruch 11, dadurch gekennzeichnet, dass $R_1$ für in 2-Stellung durch eine Gruppe —Y—$C_2$-$C_6$-Halogenalkenyl substituiertes Phenyl, $R_2$ für Wasserstoff, Z für Sauerstoff, $R_3$ für Wasserstoff oder $C_1$-$C_4$-Alkyl, $R_4$ für $C_1$-$C_4$-Alkoxy, $C_1$-$C_4$-Alkylthio, —N—di-$C_1$-$C_4$-Alkyl, —$SO_2$—$C_1$-$C_4$-Alkyl oder $C_1$-$C_4$-Halogenalkoxy und Y für Sauerstoff oder Schwefel stehen.

13. Mittel gemäss Anspruch 12, dadurch gekennzeichnet, dass $R_1$ für 2-(1,2-Dichlorvinyloxy)-phenyl oder 2-(1,2-Dichlorvinylthio)-phenyl steht.

14. Mittel gemäss Anspruch 13, dadurch gekennzeichnet, dass $R_1$ für 2-(1,2-Dichlorvinyloxy)-phenyl steht.

15. Mittel gemäss Anspruch 10, dadurch gekennzeichnet, dass $R_1$ für in 2-Stellung durch Fluor, Chlor, Nitro, $C_1$-$C_4$-Alkoxycarbonyl, $C_2$-$C_5$-Alkoxyalkoxy, $C_1$-$C_4$-Halogenalkoxy, $C_1$-$C_4$-Halogenalkylthio, $C_1$-$C_4$-Halogenalkyl oder —Y—$C_2$-$C_6$-Halogenalkenyl substituiertes Phenyl, $R_2$ für Wasserstoff, Z für Sauerstoff, $R_3$ für Wasserstoff oder $C_1$-$C_4$-Alkyl, $R_4$ für —X—$CHF_2$ und X und Y unabhängig voneinander für Sauerstoff oder Schwefel stehen.

16. Mittel gemäss Anspruch 15, dadurch gekennzeichnet, dass X Sauerstoff bedeutet.

17. Mittel gemäss Anspruch 1, dadurch gekennzeichnet, dass die Wirkstoffe ausgewählt sind aus der Reihe :

N-(2-Methoxycarbonylphenyl-sulfonyl)-N'-(1-methyl-5-difluormethoxy-1,2,4-triazol-3-yl)-harnstoff.
N-[2-(1,2-Dichlorvinyloxy)-phenyl-sulfonyl]-N'-(1,5-dimehtyl-1,2,4-triazol-3-yl)-harnstoff,
N-[2-(1,2-Dichlorvinyloxy)-phenyl-sulfonyl]-N'-(5-äthyl-1-methyl-1,2,4-triazol-3-yl)-harnstoff,
N-[2-(1,2-Dichlorvinyloxy)-phenyl-sulfonyl]-N'-(1-methyl-5-isopropyl-1,2,4-triazol-3-yl)-harnstoff,

N-[2-(1,2-Dichlorvinyloxy)-phenyl-sulfonyl]-N'-(1-methyl-5-cyclopropyl-1,2,4-triazol-3-yl)-harnstoff,
N-[2-(1,2-Dichlorvinyloxy)-phenyl-sulfonyl]-N'-(1-methyl-5-methylthio-1,2,4-triazol-3-yl)-harnstoff,
N-[2-(1,2-Dichlorvinyloxy)-phenyl-sulfonyl]-N'-(5-dimethylamino-1-methyl-1,2,4-triazol-3-yl)-harnstoff,
N-[2-(1,2-Dichlorvinyloxy)-phenyl-sulfonyl]-N'-(1-äthyl-5-methyl-1,2,4-triazol-3-yl)-harnstoff,
N-[2-(1,2-Dichlorvinyloxy)-phenyl-sulfonyl]-N'-(1,5-diäthyl-1,2,4-triazol-3-yl)-harnstoff,
N-[2-(1,2-Dichlorvinyloxy)-phenyl-sulfonyl]-N'-(5-methoxy-1-methyl-1,2,4,-triazol-3-yl)-harnstoff,
N-[2-(1,2-Dichlorvinyloxy)-phenyl-sulfonyl]-N'-(1-methyl-5-difluormethoxy-1,2,4-triazol-3-yl)-harnstoff,
N-[2-(1,2-Dichlorvinyloxy)-phenyl-sulfonyl]-N'-(1,3-dimethyl-1,2,4-triazol-5-yl)-harnstoff.

18. Verfahren zur Herstellung von Verbindungen der Formel I, dadurch gekennzeichnet, dass man ein Phenylsulfonylderivat der Formel II

$$R_1—SO_2W \qquad (II)$$

mit einer Verbindung der Formel III

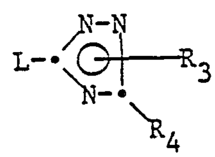

(III)

umsetzt, wobei L und W für eine der Gruppen $—NH_2—$ $—N=C=Z$ oder $—NR_2—CZ—OR$ stehen ; die Substituenten $R_1$, $R_2$, $R_3$, $R_4$ und Z die unter Formel I angegebenen Bedeutungen haben, R für einen aliphatischen oder aromatischen Rest steht ; mit der Massgabe, dass die Reaktionspartner der Formeln II und III stets so gewählt werden, dass eine Aminofunktion entweder mit Isocyanato-, Isothiocyanato- oder mit einer [$—NR_2—CZ—OR$]-Gruppe zur Reaktion gelangt.

19. Verfahren nach Anspruch 18, dadurch gekennzeichnet, dass R für $C_1$-$C_4$-Alkyl, Phenyl oder Benzyl steht.

20. Verwendung einer Verbindung der Formel I nach Anspruch 1 zur Bekämpfung von unerwünschten Pflanzensorten in Kulturen von Nutzpflanzen und/oder zur Regulierung des Pflanzenwachstums von Nutzpflanzen.

21. Verwendung nach Anspruch 20 zur selektiven Unkrautbekämpfung.

22. Verfahren zur pre- oder post-emergenten Bekämpfung von Unkräutern und/oder zur Beeinflussung des Wachstums von Nutzpflanzen, dadurch gekennzeichnet, dass man eine wirksame Menge eines Wirkstoffes der Formel I gemäss Anspruch 1 auf die Schad- oder Nutzpflanzen, deren Pflanzenteile oder deren Standort appliziert.

23. Verwendung einer Verbindung der Formel IX nach Anspruch 6 zur Bekämpfung phytopathogenen Bakterien.

24. Verwendung gemäss Anspruch 23, dadurch gekennzeichnet, dass die Bakterien zur Familie der Xanthomonoceae oder Pseudomonaceae gehören.

25. Verfahren zur Bekämpfung photopathogener Bakterien, dadurch gekennzeichnet, dass man eine wirksame Menge eines Wirkstoffes der Formel IX auf die zu schützenden Nutzpflanzen, deren Pflanzenteile oder deren Standort appliziert.

**Claims** (for the Contracting States : BE, CH, DE, FR, GB, IT, LI, NL, SE)

1. An N-arylsulfonyl-N'-triazolylurea of the formula I

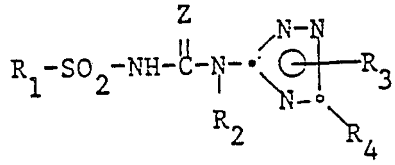

(I)

wherein

$R_1$ is a radical of the formula

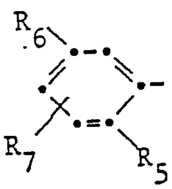

or

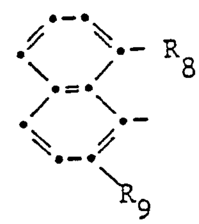

$R_2$ and $R_3$, each independently of the other, are hydrogen or $C_1$-$C_6$alkyl,

$R_4$ is hydrogen, halogen, $C_1$-$C_6$alkyl, $C_1$-$C_6$haloalkyl, cyclopropyl, —$NR_{10}R_{11}$ or—X—$R_{12}$, and

Z is oxygen or sulfur,

$R_5$ and $R_9$, each independently of the other, are hydrogen, halogen, cyano, nitro, $C_1$-$C_6$alkyl, $C_1$-$C_6$haloalkyl, $C_2$-$C_6$alkenyl which is unsubstituted or substituted by 1 to 3 halogen atoms, or are —CO—$R_{13}$ —$NR_{10}R_{11}$, —$SO_2$—$C_1$-$C_4$haloalkoxy, —$SO_2$—$NR_{10}R_{11}$, —NH—$SO_2$—$R_{15}$, —O—$SO_2$—$R_{15}$, —N($C_1$-$C_4$alkyl)—$SO_2$—$R_{15}$, or are a radical —Y—$R_{14}$ which is unsubstituted or substituted by one or more members selected from the group consisting of halogen, $C_1$-$C_4$alkoxy, $C_1$-$C_4$alkylthio, $C_1$-$C_4$alkylsulfinyl, $C_1$-$C_4$alkylsulfonyl, $C_1$-$C_4$haloalkoxy, $C_1$-$C_4$haloalkylthio, $C_1$-$C_4$haloalkylsulfinyl or $C_1$-$C_4$-haloalkylsulfonyl,

$R_6$ is hydrogen, halogen, nitro or $C_1$-$C_6$alkoxy,

$R_7$ and $R_8$, each independently of the other, are hydrogen, halogen, nitro, $C_1$-$C_6$haloalkyl, —$NH_2$, $C_1$-$C_6$alkyl, $C_1$-$C_6$alkoxy, $C_1$-$C_6$alkylthio, $C_1$-$C_6$alkylsulfinyl or $C_1$-$C_6$alkylsulfonyl,

$R_{10}$ and $R_{11}$, each independently of the other, are hydrogen, $C_1$-$C_4$alkyl, cyclopropyl, $C_2$-$C_6$alkenyl or $C_3$-$C_6$alkynyl, or $R_{10}$ and $R_{11}$ together form a $C_4$-$C_6$alkylene bridge which may be substituted by methyl or interrupted by oxygen, sulfur, —SO—, —$SO_2$—, —NH— or —N($C_1$-$C_4$) alkyl,

$R_{12}$ is $C_1$-$C_4$alkyl, $C_1$-$C_4$haloalkyl or alkoxyalkyl containing not more than 4 carbon atoms,

$R_{13}$ is hydrogen, $C_1$-$C_4$alkyl, $C_3$-$C_6$alkenyloxy, $C_3$-$C_6$alkynyloxy, $C_1$-$C_4$haloalkyl, $C_1$-$C_6$alkylthio, phenoxy, benzyloxy, —$NR_{10}R_{11}$ or $C_1$-$C_6$alkoxy which is unsubstituted or substituted by 1 to 3 halogen atoms,

$R_{14}$ is $C_1$-$C_6$alkyl, $C_2$-$C_6$alkenyl or $C_3$-$C_6$alkynyl,

$R_{15}$ is $C_1$-$C_6$alkyl or $C_1$-$C_6$haloalkyl, and

X and Y, each independently of the other, are oxygen, sulfur, a sulfinyl or sulfonyl bridge,

or a salt thereof ; with the proviso that

a) $R_1$ only has the given meaning if $R_4$ is the —X—$CHF_2$ group, and

b) $R_4$ only has the given meaning if $R_1$ is the substituted phenyl nucleus and $R_5$ is the —Y—$C_2$-$C_6$haloalkyl group.

2. A compound according to claim 1, wherein

$R_1$ is a radical of the formula

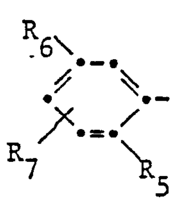

or

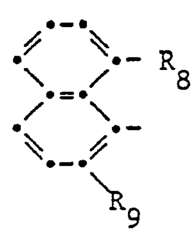

$R_2$ and $R_3$, each independently of the other, are hydrogen or $C_1$-$C_4$alkyl,

$R_4$ is hydrogen, halogen, $C_1$-$C_4$alkyl, $C_1$-$C_4$haloalkyl, cyclopropyl, —$NR_{10}R_{11}$ or —X—$R_{12}$, and

Z is oxygen or sulfur,

$R_5$ is hydrogen, halogen, cyano, nitro, $C_1$-$C_4$alkyl, $C_1$-$C_4$haloalkyl, $C_2$-$C_6$alkenyl, —CO—$R_{13}$ —$NR_{10}R_{11}$, or is a radical —Y—$R_{14}$ which is unsubstituted or substituted by one or more members selected from the group consisting of halogen, $C_1$-$C_4$alkoxy, $C_1$-$C_4$alkylthio, $C_1$-$C_4$alkylsulfinyl, $C_1$-$C_4$alkylsulfonyl, $C_1$-$C_4$haloalkoxy, $C_1$-$C_4$haloalkylthio, $C_1$-$C_4$haloalkylsulfinyl or $C_1$-$C_4$haloalkylsulfonyl,

$R_6$ is hydrogen, halogen, nitro or $C_1$-$C_4$alkoxy,

$R_7$ is hydrogen, halogen, nitro, $C_1$-$C_4$haloalkyl, —$NH_2$, $C_1$-$C_4$alkyl, $C_1$-$C_4$alkoxy, $C_1$-$C_3$alkylthio, $C_1$-$C_3$alkylsulfinyl or $C_1$-$C_3$alkylsulfonyl,

$R_{10}$ and $R_{11}$, each independently of the other, are hydrogen, $C_1$-$C_4$alkyl, cyclopropyl, $C_2$-$C_6$alkenyl or $C_3$-$C_6$alkynyl, or $R_{10}$ and $R_{11}$ together form a $C_4$-$C_6$alkylene bridge which may be substituted by methyl or interrupted by oxygen, sulfur, —SO—, —$SO_2$—, —NH— or —N($C_1$-$C_4$)alkyl,

$R_{12}$ is $C_1$-$C_4$alkyl, $C_1$-$C_4$haloalkyl or alkoxyalkyl containing not more than 4 carbon atoms,

$R_{13}$ is hydrogen, $C_1$-$C_4$alkyl, $C_3$-$C_5$alkenyloxy, $C_3$-$C_5$alkynyloxy, $C_1$-$C_5$alkylthio, phenoxy, benzyloxy, —$NR_{10}R_{11}$ or $C_1$-$C_5$alkoxy which is unsubstituted or substituted by 1 to 3 halogen atoms,

$R_{14}$ is $C_1$-$C_6$alkyl, $C_2$-$C_6$alkenyl or $C_3$-$C_6$alkynyl, and

X and Y, each independently of the other, are oxygen, sulfur, a sulfinyl or sulfonyl bridge, and $R_8$ has the same meaning as $R_7$ and $R_9$ has the same meaning as $R_5$,

or a salt thereof ; with the proviso that

a) $R_1$ only has the given meaning if $R_4$ is the —X—$CHF_2$ group, and

b) $R_4$ only has the given meaning if $R_1$ is the substituted phenyl nucleus and $R_5$ is the —Y—$C_2$-$C_6$-haloalkyl group

3. A compound of the formula I according to claim 1, wherein $R_1$ is the group

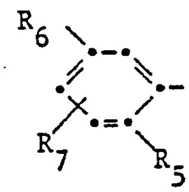

wherein $R_5$ is the —Y—$C_2$-$C_6$haloalkenyl group and $R_6$ and $R_7$ are as defined for formula I.

4. A compound according to claim 1, wherein $R_4$ is the —X—$CHF_2$ group.

5. A compound according to claim 3 of the formula IX

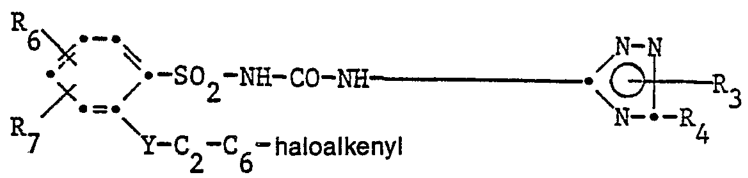

(IX)

wherein $R_3$ is hydrogen or $C_1$-$C_4$alkyl, $R_4$ is $C_1$-$C_4$alkoxy, $C_1$-$C_4$alkylthio, —N—di($C_1$-$C_4$)alkyl, —$SO_2$—$C_1$-$C_4$alkyl or $C_1$-$C_4$haloalkoxy, and Y is oxygen or sulfur and $R_6$ and $R_7$ are as defined for formula I in claim 1.

6. A compound according to claim 5, wherein $R_6$ and $R_7$ are hydrogen.

7. A compound according to claim 6, wherein the —Y—$C_2$-$C_6$haloalkenyl group is 1,2-dichlorovinyloxy or 1,2-dichlorovinylthio.

8. A compound according to claim 7, wherein the —Y—$C_2$-$C_6$haloalkenyl group is 1,2-dichlorovinyloxy.

9. A compound according to claim 1, wherein $R_1$ is phenyl substituted in the 2-position by fluorine, chlorine, nitro, $C_1$-$C_4$alkoxycarbonyl, $C_2$-$C_5$alkoxyalkoxy, $C_1$-$C_4$haloalkoxy, $C_1$-$C_4$haloalkylthio, $C_1$-$C_4$haloalkyl or —Y—$C_2$-$C_6$haloalkenyl, $R_2$ is hydrogen, Z is oxygen, $R_3$ is hydrogen or $C_1$-$C_4$alkyl, $R_4$ is —X—$CHF_2$, and each of X and Y independently of the other is oxygen or sulfur.

10. A compound according to claim 1 of the formula Ia

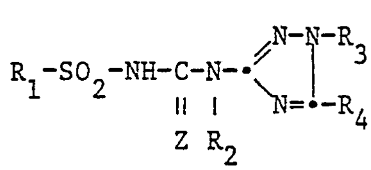

(Ia)

wherein $R_1$ to $R_4$ and Z are as defined for formula I in claim 1.

11. A compound according to claim 10, wherein $R_1$ is phenyl substituted in the 2-position by a —Y—$C_2$-$C_6$haloalkenyl group, $R_2$ is hydrogen, Z is oxygen, $R_3$ is hydrogen or $C_1$-$C_4$alkyl, $R_4$ is $C_1$-$C_4$alkoxy, $C_1$-$C_4$alkylthio, —N—di($C_1$-$C_4$)alkyl, —$SO_2$—$C_1$-$C_4$alkyl or $C_1$-$C_4$haloalkoxy and Y is oxygen or sulfur.

12. A compound according to claim 11, wherein $R_1$ is 2-(1,2-dichlorovinyloxy)phenyl or 2-(1,2-dichlorovinylthio)phenyl.

13. A compound according to claim 12, wherein $R_1$ is 2-(1,2-dichlorovinyloxy)phenyl.

14. A compound according to claim 10, wherein $R_1$ is phenyl substituted in the 2-position by fluorine, chlorine, nitro, $C_1$-$C_4$alkoxycarbonyl, $C_2$-$C_5$alkoxyalkoxy, $C_1$-$C_4$haloalkoxy, $C_1$-$C_4$haloalkylthio, $C_1$-$C_4$haloalkyl or —Y—$C_2$-$C_6$haloalkenyl, $R_2$ is hydrogen, Z is oxygen, $R_3$ is hydrogen or $C_1$-$C_4$alkyl, $R_4$ is —X—$CHF_2$, and each of X and Y independently of the other is oxygen or sulfur.

15. A compound according to claim 14, wherein X is oxygen.

16. A compound of the formula I according to claim 1, selected from the group consisting of

N-(2-methoxycarbonylphenylsulfonyl)-N'-(1-methyl-5-difluoromethoxy-1,2,4-triazol-3-yl)urea,
N-[2-(1,2-dichlorovinyloxy)phenylsulfonyl]-N'-(1,5-dimethyl-1,2,4-triazol-3-yl)urea,
N-[2-(1,2-dichlorovinyloxy)phenylsulfonyl]-N'-(5-ethyl-1-methyl-1,2,4-triazol-3-yl)urea,
N-[2-(1,2-dichlorovinyloxy)phenylsulfonyl]-N'-(1-methyl-5-isopropyl-1,2,4-triazol-3-yl)urea,
N-[2-(1,2-dichlorovinyloxy)phenylsulfonyl]-N'-(1-methyl-5-cyclopropyl-1,2,4-triazol-3-yl)urea,
N-[2-(1,2-dichlorovinyloxy)phenylsulfonyl]-N'-(1-methyl-5-methylthio-1,2,4-triazol-3-yl)urea,
N-[2-(1,2-dichlorovinyloxy)phenylsulfonyl]-N'-(5-dimethylamino-1-methyl-1,2,4-triazol-3-yl)urea,
N-[2-(1,2-dichlorovinyloxy)phenylsulfonyl]-N'-(1-ethyl-5-methyl-1,2,4-triazol-3-yl)urea,
N-[2-(1,2-dichlorovinyloxy)phenylsulfonyl]-N'-(1,5-diethyl-1,2,4-triazol-3-yl)urea,
N-[2-(1,2-dichlorovinyloxy)phenylsulfonyl]-N'-(5-methoxy-1-methyl-1,2,4-triazol-3-yl)urea,
N-[2-(1,2-dichlorovinyloxy)phenylsulfonyl]-N'-(1-methyl-5-difluormethoxy-1,2,4-triazol-3-yl)urea,
N-[2-(1,2-dichlorovinyloxy)phenylsulfonyl]-N'-(1,3-dimethyl-1,2,4-triazol-5-yl)urea.

17. A process for the preparation of a compound of the formula I, which comprises reacting a phenylsulfonyl derivative of the formula II

$$R_1—SO_2W \qquad (II)$$

with a compound of the formula III

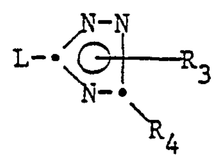

(III)

wherein L and W are a $—NH_2$, $—N=C=Z$ or $—NR_2—CZ—OR$ group, the substituents $R_1$, $R_2$, $R_3$, $R_4$ and Z are as defined for formula I, R is an aliphatic or aromatic radical, with the proviso that the reactants of the formulae II and III are always chosen such that an amino function reacts either with an isocyanato, isothiocyanato or an [$—NR_2—CZ—OR$] group.

18. A process according to claim 17, wherein R is $C_1$-$C_4$alkyl, phenyl or benzyl.

19. A herbicidal, bactericidal and/or plant growth regulating composition which contains at least one compound of the formula I as claimed in claim 1 as active component.

20. A composition according to claim 19, which contains 0.1 to 95 % by weight of a compound of the formula I as claimed in claim 1, 1 to 99 % by weight of a solid or liquid adjuvant, and 0.1 to 25 % by weight of a surfactant.

21. Use of a compound of formula I according to claim 1 for controlling undesirable plants in crops of useful plants and/or for regulating plant growth.

22. Use according to claim 21 for selective weed control.

23. A method of selectively controlling weeds pre- or postemergence and/or of influencing the growth of useful plants, which comprises applying to the noxious or useful plants, or to the locus thereof, an effective amount of a compound of the formula I as claimed in claim 1.

24. Use of a compound of formula IX according to claim 5 for controlling phytopathogenic bacteria.

25. Use according to claim 24, wherein the bacteria to be controlled are bacteria of the families Xanthomonoceae or Pseudomonaceae.

26. A method of controlling phytopathogenic bacteria, which comprises applying to the useful plants which is desired to protect, to parts thereof or to the locus thereof, an effective amount of a compound of the formula IX.

**Claims** (for the Contracting State AT)

1. A herbicidal and/or plant growth regulating composition containing as at least one active component an N-arylsulfonyl-N'-triazolylurea of the formula I

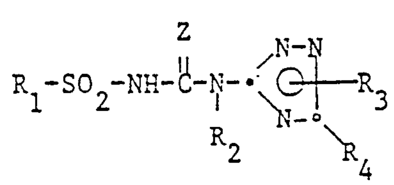

(I)

wherein

$R_1$ is a radical of the formula

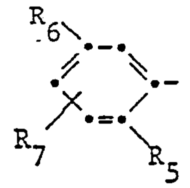

or

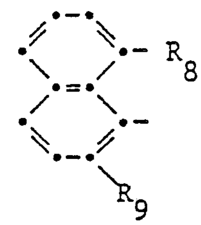

$R_2$ and $R_3$, each independently of the other, are hydrogen or $C_1$-$C_6$alkyl,

$R_4$ is hydrogen, halogen, $C_1$-$C_6$alkyl, $C_1$-$C_6$haloalkyl, cyclopropyl, —$NR_{10}R_{11}$ or —X—$R_{12}$, and

Z is oxygen or sulfur,

$R_5$ and $R_9$, each independently of the other, are hydrogen, halogen, cyano, nitro, $C_1$-$C_6$alkyl, $C_1$-$C_6$haloalkyl, $C_2$-$C_6$alkenyl which is unsubstituted or substituted by 1 to 3 halogen atoms, or are —CO—$R_{13}$ —$NR_{10}R_{11}$, —$SO_2$—$C_1$-$C_4$haloalkoxy, —$SO_2$—$NR_{10}R_{11}$, —NH—$SO_2$—$R_{15}$, —O—$SO_2$—$R_{15}$, —N($C_1$-$C_4$alkyl)—$SO_2$—$R_{15}$, or are a radical —Y—$R_{14}$ which is unsubstituted or substituted by one or more members selected from the group consisting of halogen, $C_1$-$C_4$alkoxy, $C_1$-$C_4$alkylthio, $C_1$-$C_4$alkylsulfinyl, $C_1$-$C_4$alkylsulfonyl, $C_1$-$C_4$haloalkoxy, $C_1$-$C_4$haloalkylthio, $C_1$-$C_4$haloalkylsulfinyl or $C_1$-$C_4$haloalkylsulfonyl,

$R_6$ is hydrogen, halogen, nitro or $C_1$-$C_6$alkoxy,

$R_7$ and $R_8$, each independently of the other, are hydrogen, halogen, nitro, $C_1$-$C_6$haloalkyl, —$NH_2$, $C_1$-$C_6$alkyl, $C_1$-$C_6$alkoxy, $C_1$-$C_6$alkylthio, $C_1$-$C_6$-alkylsulfinyl or $C_1$-$C_6$alkylsulfonyl,

$R_{10}$ and $R_{11}$, each independently of the other, are hydrogen, $C_1$-$C_4$alkyl, cyclopropyl, $C_2$-$C_6$alkenyl or $C_3$-$C_6$alkynyl, or $R_{10}$ and $R_{11}$ together form a $C_4$-$C_6$alkylene bridge which may be substituted by methyl or interrupted by oxygen, sulfur, —SO—, —$SO_2$—, —NH— or —N($C_1$-$C_4$)alkyl,

$R_{12}$ is $C_1$-$C_4$alkyl, $C_1$-$C_4$haloalkyl or alkoxyalkyl containing not more than 4 carbon atoms,

$R_{13}$ is hydrogen, $C_1$-$C_4$alkyl, $C_3$-$C_6$alkenyloxy, $C_3$-$C_6$alkynyloxy, $C_1$-$C_4$-haloalkyl, $C_1$-$C_6$alkylthio, phenoxy, benzyloxy, —$NR_{10}R_{11}$ or $C_1$-$C_6$alkoxy which is unsubstituted or substituted by 1 to 3 halogen atoms,

$R_{14}$ is $C_1$-$C_6$alkyl, $C_2$-$C_6$alkenyl or $C_3$-$C_6$alkynyl,

$R_{15}$ is $C_1$-$C_6$alkyl or $C_1$-$C_6$haloalkyl, and

X and Y, each independently of the other, are oxygen, sulfur, a sulfinyl or sulfonyl bridge ; with the proviso that

a) $R_1$ only has the given meaning if $R_4$ is the —X—$CHF_2$ group, and

b) $R_4$ only has the given meaning if $R_1$ is the substituted phenyl nucleus and $R_5$ is the —Y—$C_2$-$C_6$haloalkyl group.

2. A composition according to claim 1, which contains 0.1 to 95 % by weight of a compound of the formula I as claimed in claim 1, 1 to 99 % by weight of a solid or liquid adjuvant, and 0.1 to 25 % by weight of a surfactant.

3. A composition according to claim 1, wherein

$R_1$ is a radical of the formula

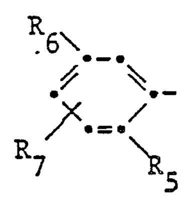

or

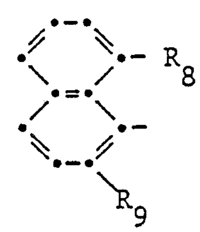

$R_2$ and $R_3$, each independently of the other, are hydrogen or $C_1$-$C_4$alkyl,

$R_4$ is hydrogen, halogen, $C_1$-$C_4$alkyl, $C_1$-$C_4$haloalkyl, cyclopropyl, —$NR_{10}R_{11}$ or —X—$R_{12}$, and

Z is oxygen or sulfur,

$R_5$ is hydrogen, halogen, cyano, nitro, $C_1$-$C_4$alkyl, $C_1$-$C_4$haloalkyl, $C_2$-$C_6$alkenyl, —CO—$R_{13}$ —$NR_{10}R_{11}$, or is a radical —Y—$R_{14}$ which is unsubstituted or substituted by one or more members selected from the group consisting of halogen, $C_1$-$C_4$alkoxy, $C_1$-$C_4$alkylthio, $C_1$-$C_4$-alkylsulfinyl, $C_1$-$C_4$alkylsulfonyl, $C_1$-$C_4$haloalkoxy, $C_1$-$C_4$haloalkylthio, $C_1$-$C_4$haloalkylsulfinyl or $C_1$-$C_4$haloalkylsulfonyl,

$R_6$ is hydrogen, halogen, nitro or $C_1$-$C_4$alkoxy,

$R_7$ is hydrogen, halogen, nitro, $C_1$-$C_4$haloalkyl, —$NH_2$, $C_1$-$C_4$alkyl, $C_1$-$C_4$alkoxy, $C_1$-$C_3$alkylthio, $C_1$-$C_3$alkylsulfinyl or $C_1$-$C_3$alkylsulfonyl,

$R_{10}$ and $R_{11}$, each independently of the other, are hydrogen, $C_1$-$C_4$alkyl, cyclopropyl, $C_2$-$C_6$alkenyl or $C_3$-$C_6$alkynyl, or $R_{10}$ and $R_{11}$ together form a $C_4$-$C_6$alkylene bridge which may be substituted by methyl or interrupted by oxygen, sulfur, —SO—, —$SO_2$—, —NH— or —N($C_1$-$C_4$)alkyl,

$R_{12}$ is $C_1$-$C_4$alkyl, $C_1$-$C_4$haloalkyl or alkoxyalkyl containing not more than 4 carbon atoms,

$R_{13}$ is hydrogen, $C_1$-$C_4$alkyl, $C_3$-$C_5$alkenyloxy, $C_3$-$C_5$alkynyloxy, $C_1$-$C_5$-alkylthio, phenoxy, benzyloxy, —$NR_{10}R_{11}$ or $C_1$-$C_5$alkoxy which is unsubstituted or substituted by 1 to 3 halogen atoms,

$R_{14}$ is $C_1$-$C_6$alkyl, $C_2$-$C_6$alkenyl or $C_3$-$C_6$alkynyl, and

X and Y, each independently of the other, are oxygen, sulfur, a sulfinyl or sulfonyl bridge, and $R_8$ has the same meaning as $R_7$ and $R_9$ has the same meaning as $R_5$ ; with the proviso that

a) $R_1$ only has the given meaning if $R_4$ is the —X—$CHF_2$ group, and

b) $R_4$ only has the given meaning if $R_1$ is the substituted phenyl nucleus and $R_5$ is the —Y—$C_2$-$C_6$haloalkyl group.

4. A composition according to claim 1, wherein $R_1$ is the group

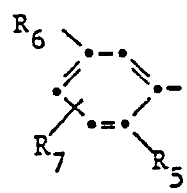

wherein $R_5$ is the —Y—$C_2$-$C_6$haloalkenyl group and $R_6$ and $R_7$ are as defined for formula I.

5. A composition according to claim 1, wherein $R_4$ is the —X—$CHF_2$ group.

6. A composition according to claim 4 containing a compound of the formula IX

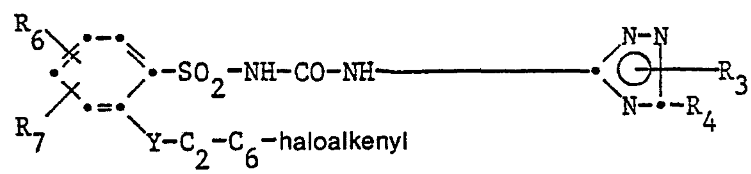

(IX)

wherein $R_3$ is hydrogen or $C_1$-$C_4$alkyl, $R_4$ is $C_1$-$C_4$alkoxy, $C_1$-$C_4$alkylthio, —N—di($C_1$-$C_4$)alkyl, —$SO_2$—$C_1$-$C_4$alkyl or $C_1$-$C_4$haloalkoxy, and Y is oxygen or sulfur and $R_6$ and $R_7$ are as defined for formula I in claim 1.

7. A composition according to claim 6, wherein $R_6$ and $R_7$ are hydrogen.

8. A composition according to claim 7, wherein the —Y—$C_2$-$C_6$haloalkenyl group is 1,2-dichlorovinyloxy or 1,2-dichlorovinylthio.

9. A composition according to claim 8, wherein the —Y—$C_2$-$C_6$haloalkenyl group is 1,2-dichlorovinyloxy.

10. A composition according to claim 1, wherein $R_1$ is phenyl substituted in the 2-position by fluorine, chlorine, nitro, $C_1$-$C_4$alkoxycarbonyl, $C_2$-$C_5$alkoxyalkoxy, $C_1$-$C_4$haloalkoxy, $C_1$-$C_4$haloalkylthio, $C_1$-$C_4$haloalkyl or —Y—$C_2$-$C_6$haloalkenyl, $R_2$ is hydrogen, Z is oxygen, $R_3$ is hydrogen or $C_1$-$C_4$alkyl, $R_4$ is —X—$CHF_2$, and each of X and Y independently of the other is oxygen or sulfur.

11. A composition according to claim 1 containing a compound of the formula Ia

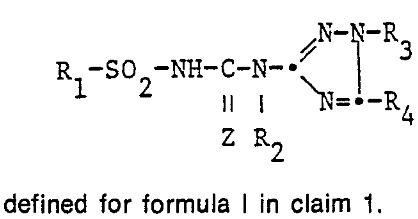

(Ia)

wherein $R_1$ to $R_4$ and Z are as defined for formula I in claim 1.

12. A composition according to claim 11, wherein $R_1$ is phenyl substituted in the 2-position by a —Y—$C_2$-$C_2$haloalkenyl group, $R_2$ is hydrogen, Z is oxygen, $R_3$ is hydrogen or $C_1$-$C_4$alkyl, $R_4$ is $C_1$-$C_4$alkoxy, $C_1$-$C_4$alkylthio, —N—di($C_1$-$C_4$)alkyl, —$SO_2$—$C_1$-$C_4$alkyl or $C_1$-$C_4$haloalkoxy and Y is oxygen or sulfur.

13. A composition according to claim 12, wherein $R_1$ is 2-(1,2-dichlorovinyloxy)phenyl or 2-(1,2-dichlorovinylthio)phenyl.

14. A composition according to claim 13, wherein $R_1$ is 2-(1,2-dichlorovinyloxy)phenyl.

15. A composition according to claim 11, wherein $R_1$ is phenyl substituted in the 2-position by fluorine, chlorine, nitro, $C_1$-$C_4$alkoxycarbonyl, $C_2$-$C_5$alkoxyalkoxy, $C_1$-$C_4$haloalkoxy, $C_1$-$C_4$haloalkylthio, $C_1$-$C_4$haloalkyl or —Y—$C_2$-$C_6$haloalkenyl, $R_2$ is hydrogen, Z is oxygen, $R_3$ is hydrogen or $C_1$-$C_4$alkyl, $R_4$ is —X—$CHF_2$, and each of X and Y independently of the other is oxygen or sulfur.

16. A composition according to claim 15, wherein X is oxygen.

17. A composition according to claim 1, containing a compound selected from the group consisting of

N-(2-methoxycarbonylphenylsulfonyl)-N'-(1-methyl-5-difluoromethoxy-1,2,4-triazol-3-yl)urea,
N-[2-(1,2-dichlorovinyloxy)phenylsulfonyl]-N'-(1,5-dimethyl-1,2,4-triazol-3-yl)urea,
N-[2-(1,2-dichlorovinyloxy)phenylsulfonyl]-N'-(5-ethyl-1-methyl-1,2,4-triazol-3-yl)urea,
N-[2-(1,2-dichlorovinyloxy)phenylsulfonyl]-N'-(1-methyl-5-isopropyl-1,2,4-triazol-3-yl)urea,
N-[2-(1,2-dichlorovinyloxy)phenylsulfonyl]-N'-(1-methyl-5-cyclopropyl-1,2,4-triazol-3-yl)urea,
N-[2-(1,2-dichlorovinyloxy)phenylsulfonyl]-N'-(1-methyl-5-methylthio-1,2,4-triazol-3-yl)urea,
N-[2-(1,2-dichlorovinyloxy)phenylsulfonyl]-N'-(5-dimethylamino-1-methyl-1,2,4-triazol-3-yl)urea,
N-[2-(1,2-dichlorovinyloxy)phenylsulfonyl]-N'-(1-ethyl-5-methyl-1,2,4-triazol-3-yl)urea,
N-[2-(1,2-dichlorovinyloxy)phenylsulfonyl]-N'-(1,5-diethyl-1,2,4-triazol-3-yl)urea,
N-[2-(1,2-dichlorovinyloxy)phenylsulfonyl]-N'-(5-methoxy-1-methyl-1,2,4-triazol-3-yl)urea,
N-[2-(1,2-dichlorovinyloxy)phenylsulfonyl]-N'-(1-methyl-5-difluormethoxy-1,2,4-triazol-3-yl)urea, and
N-[2-(1,2-dichlorovinyloxy)phenylsulfonyl]-N'-(1,3-dimethyl-1,2,4-triazol-5-yl)urea.

18. A process for the preparation of a compound of the formula I, which comprises reacting a phenylsulfonyl derivative of the formula II

$$R_1—SO_2W \quad (II)$$

with a compound of the formula III

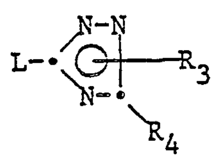

(III)

wherein L and W are a —$NH_2$, —N=C=Z or —$NR_2$—CZ—OR group, the substituents $R_1$, $R_2$, $R_3$, $R_4$ and Z are as defined for formula I, R is an aliphatic or aromatic radical, with the proviso that the reactants of the formulae II and III are always chosen such that an amino function reacts either with an isocyanato, isothiocyanato or an [—$NR_2$—CZ—OR] group.

19. A process according to claim 18, wherein R is $C_1$-$C_4$alkyl, phenyl or benzyl.

20. Use of compound of formula I according to claim 1 for controlling undesirable plants in crops of useful plants and/or for regulating plant growth.

21. Use according to claim 20 for selective weed control.

22. A method of selectively controlling weeds pre- or postemergence and/or influencing the growth of useful plants, which comprises applying to the noxious or useful plants, or to the locus thereof, an effective amount of a compound of the formula I as claimed in claim 1.

23. Use of a compound of formula IX according to claim 6 for controlling phytopathogenic bacteria.

24. Use according to claim 23, wherein the bacteria to be controlled are bacteria of the families Xanthomonoceae or Pseudomonaceae.

25. A method of controlling phytopathogenic bacteria, which comprises applying to the useful plants which is desired to protect, to parts thereof or to the locus thereof, an effective amount of a compound of the formula IX.

**Revendications** (pour les Etats contractants : BE, CH, DE, FR, GB, IT, LI, NL, SE)

1. N-arylsulfonyl-N'-triazolyl-urée de formule I

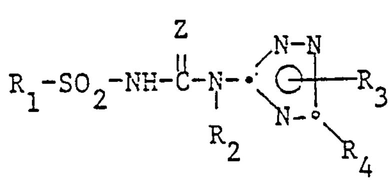

(I)

où

$R_1$ représente un radical de formule

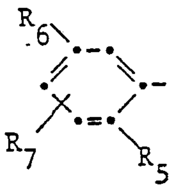

ou

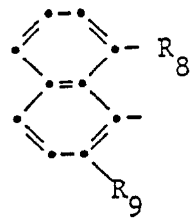

$R_2$ et $R_3$ représentent indépendamment l'un de l'autre un hydrogène ou un alcoyle en $C_1$ à $C_6$

$R_4$ représente un hydrogène, halogène, alcoyle en $C_1$ à $C_6$, halogène-alcoyle en $C_1$ à $C_6$, cyclopropyle, $—NR_{10}R_{11}$ ou $—X—R_{12}$ et

Z représente un oxygène ou un soufre, ou

$R_5$ et $R_9$ représentent un hydrogène, halogène, cyano, nitro, alcoyle en $C_1$ à $C_6$, halogène-alcoyle en $C_1$ à $C_6$, alcényle en $C_2$ à $C_6$ éventuellement substitué par de 1 à 3 atomes d'halogène, $—CO—R_{13}$, $—NR_{10}R_{11}$, $—SO_2—C_1$-$C_4$-halogènealcoxy, $—SO_2—NR_{10}R_{11}$, $—NH—SO_2—R_{15}$, N(C alcoyle en $C_1$ à $C_4$), ou un radical $—Y—R_{14}$ éventuellement substitué par un ou plusieurs substituants, de la série halogène, alcoxy en $C_1$ à $C_4$, alcoyl-thio en $C_1$ à $C_4$, alcoyl-sulfinyle en $C_1$ à $C_4$, alcoyl-sulfonyle en $C_1$ à $C_4$, halogène-alcoxy en $C_1$ à $C_4$, halogène-alcoyl-thio en $C_1$ à $C_4$, halogène-alcoyl-sulfinyle en $C_1$ à $C_4$ ou halogène-sulfonyle en $C_1$ à $C_4$,

$R_6$ représente un hydrogène, halogène, nitro ou alcoxy en $C_1$ à $C_6$,

$R_7$ et $R_8$ représentent un hydrogène, halogène, nitro, halogène-alcoyle en $C_1$ à $C_6$, $—NH_2$, alcoyle en $C_1$ à $C_6$, alcoxy en $C_1$ à $C_6$, alcoylthio en $C_1$ à $C_6$, alcoyl-sulfonyle en $C_1$ à $C_6$ ou alcoyl-sulfonyle en $C_1$ à $C_6$,

$R_{10}$ et $R_{11}$ représentent indépendamment l'un de l'autre un hydrogène, alcoyle en $C_1$ à $C_4$ ou cyclopropyle ou $R_{10}$ et $R_{11}$ représentent ensemble un pont alcoylène éventuellement substitué par un méthyle ou interrompu par un oxygène, soufre, —SO—, $—SO_2—$, —NH— ou —N(alcoyle en $C_1$ à $C_4$),

$R_{12}$ représente un alcoyle en $C_1$ à $C_4$, halogènealcoyle en $C_1$ à $C_4$ ou alcoxy-alcoyle avec au plus 4 atomes de carbone,

$R_{13}$ représente un hydrogène, alcoyle en $C_1$ à $C_4$, alcényloxy en $C_3$ à $C_6$, alcynyloxy en $C_3$ à $C_6$, halogène-alcoyle en $C_1$ à $C_4$, alcoylthio en $C_1$ à $C_6$, phénoxy, benzyloxy, $—NR_{10}R_{11}$ ou alcoxy en $C_1$ à $C_6$ éventuellement substitué par 1 à 3 atomes d'halogène,

$R_{14}$ représente un alcoyle en $C_1$ à $C_6$, alcényle en $C_2$ à $C_6$ ou alcynyle en $C_3$ à $C_6$,

$R_{15}$ représente un alcoyle en $C_1$ à $C_6$ ou un halogène-alcoyle en $C_1$ à $C_6$ et

X et Y représente indépendamment l'un de l'autre un oxygène, soufre, un pont sulfinyle ou sulfonyle, ainsi que les sels de ces composés ;
avec la précision que

a) $R_1$ n'a la signification indiquée que lorsque $R_4$ représente le groupe $—X—CHF_2$, et

b) $R_4$ n'a la signification indiquée que lorsque $R_1$ représente le noyau phényle substitué et $R_5$ représente le groupe —Y—halogène-alcényle en $C_2$ à $C_6$.

2. Composés selon la revendication 1, caractérisés en ce que

$R_1$ représente un radical de formule

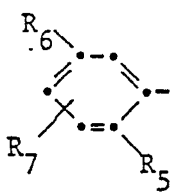

ou

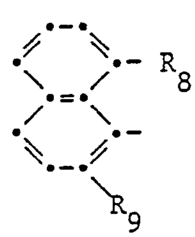

$R_2$ et $R_3$ représentent indépendamment l'un de l'autre un hydrogène ou un alcoyle en $C_1$ à $C_4$

$R_4$ représente un hydrogène, halogène, alcoyle en $C_1$ à $C_4$, halogène-alcoyle en $C_1$ à $C_4$, cyclopropyle, $—NR_{10}R_{11}$ ou $—X—R_{12}$ et

Z représente un oxygène ou un soufre,

où

$R_5$ représente un hydrogène, halogène, cyano, nitro, alcoyle en $C_1$ à $C_4$, halogène-alcoyle en $C_1$ à $C_4$, alcényle en $C_2$ à $C_6$, $—CO—R_{13}$, $—NR_{10}R_{11}$ ou un radical $—Y—R_{14}$ éventuellement substitué par un ou

plusieurs substituants de la série halogène, alcoxy en $C_1$ à $C_4$, alcoylthio en $C_1$ à $C_4$, alcoyl-sulfinyle en $C_1$ à $C_4$, alcoyl-sulfonyle en $C_1$ à $C_4$, halogène-alcoxy en $C_1$ à $C_4$, halogène-alcoyl-thio en $C_1$ à $C_4$, halogène-alcoyl-sulfinyle en $C_1$ à $C_4$ ou halogène-sulfonyle en $C_1$ à $C_4$,

$R_6$ représente un hydrogène, halogène, nitro ou alcoxy en $C_1$ à $C_4$,

$R_7$ représente un hydrogène, halogène, nitro, halogène-alcoyle en $C_1$ à $C_4$, —$NH_2$, alcoyle en $C_1$ à $C_4$, alcoxy en $C_1$ à $C_4$, alcoyl-thio en $C_1$ à $C_3$, alcoyl-sufinyle en $C_1$ à $C_3$ ou alcoyle sulfonyle en $C_1$ à $C_3$,

$R_{10}$ et $R_{11}$ représentent indépendamment l'un de l'autre un hydrogène, alcoyle en $C_1$ à $C_3$, cyclopropyle, alcényle en $C_2$ à $C_6$ ou alcynyle en $C_3$ à $C_6$ ou $R_{10}$ et $R_{11}$ représentent ensemble un pont alcoylène en $C_4$ à $C_6$ éventuellement substitué par un méthyle ou interrompu par un méthylène, soufre, —SO—, —$SO_2$—, —NH— ou —N(alcoyle en $C_1$ à $C_4$),

$R_{12}$ représente un alcoyle en $C_1$ à $C_4$, halogène-alcoyle en $C_1$ à $C_4$ ou alcoxy-alcoyle avec au plus 4 atomes de carbone,

$R_{13}$ représente un hydrogène, alcoyle en $C_1$ à $C_4$, alcényloxy en $C_3$ à $C_5$, alcynyloxy en $C_3$ à $C_5$, alcoyl-thio en $C_1$ à $C_5$, phénoxy, benzyloxy, —$NR_{10}R_{11}$ ou alcoxy en $C_1$ à $C_5$ éventuellement substitué par de 1 à 3 atomes d'halogène,

$R_{14}$ représente un alcoyle en $C_1$ à $C_6$, alcényle en $C_2$ à $C_6$ ou alcynyle en $C_3$ à $C_6$ et

X et Y représentent indépendamment l'un de l'autre un oxygène, soufre, un pont sulfinyle ou un pont sulfonyle, où $R_8$ a la même signification que $R_7$ et $R_9$ a la même signification que $R_5$ ;

ainsi que les sels de ces composés, avec cette précision que

a) $R_1$ n'a la signification indiquée que lorsque $R_4$ représente le groupe —X—$CHF_2$, et que

b) $R_4$ n'a la signification indiquée que lorsque $R_1$ représente le noyau phényle substitué et $R_5$ représente le groupe —Y—halogène-alcényle en $C_2$ à $C_6$.

3. Composés de formule I selon la revendication I, caractérisés en ce que le substituant $R_1$ représente le groupe

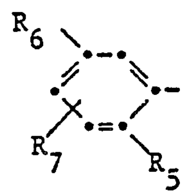

où $R_5$ représente le groupe —Y—halogène-alcoyle en $C_2$ à $C_6$ et les substituants ont les significations données pour la formule I.

4. Composés selon la revendication 1, caractérisés en ce que $R_4$ représente le groupe —X—$CHF_2$.

5. Composés selon la revendication 3, caractérisés en ce qu'ils correspondent à la formule IX

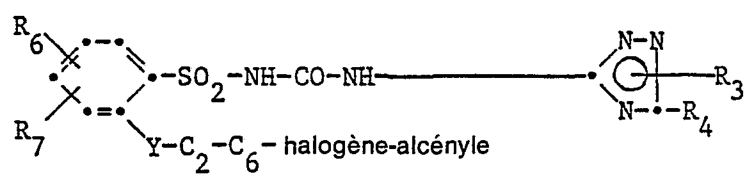

(IX)

où $R_3$ représente un hydrogène ou un alcoyle en $C_1$ à $C_4$, $R_4$ représente un alcoxy en $C_1$ à $C_4$, alcoyl-thio en $C_1$ à $C_4$, —N—di-alcoyle en $C_1$ à $C_4$, —$SO_2$—alcoyle en $C_1$ à $C_4$ ou halogène-alcoxy en $C_1$ à $C_4$ et Y représente un oxygène ou un soufre et $R_6$ et $R_7$ ont la signification donnée sous la formule I dans la revendication I.

6. Composés selon la revendication 5, caractérisés en ce que $R_6$ et $R_7$ représentent un hydrogène.

7. Composés selon la revendication 6, caractérisés en ce que le groupe —Y—halogène-alcényle en $C_2$ à $C_6$ représente un 1,2-dichlorovinyloxy ou 1,2-dichlorovinyl-thio.

8. Composés selon la revendication 7, caractérisés en ce que le groupe —Y—halogène-alcényle en $C_2$ à $C_6$ représente un 1,2-dichlorovinyloxy.

9. Composés selon la revendication 1, caractérisés en ce que $R_1$ représente un phényle substitué en position 2 par un fluor, chlore, nitro, alcoxycarbonyle en $C_1$ à $C_4$, alcoxy-alcoxy en $C_2$ à $C_5$, halogène-alcoxy en $C_1$ à $C_4$, halogène-alcoyl-thio en $C_1$ à $C_4$, halogène-alcoxy en $C_1$ à $C_4$ ou —Y—halogène-alcényle en $C_2$ à $C_6$, $R_2$ représente un hydrogène, Z représente un oxygène, $R_3$ représente un hydrogène ou un alcoyle en $C_1$ à $C_4$, $R_4$ représente —X—$CHF_2$ et X et Y représentent indépendamment l'un de l'autre un oxygène ou un soufre.

10. Composés selon la revendication 1, caractérisés en ce qu'ils correspondent à la formule Ia

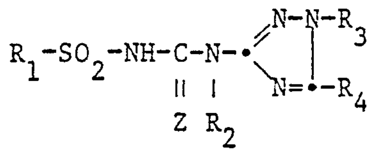

(Ia)

où $R_1$ à $R_4$ et Z ont la signification donnée sous la formule I dans la revendication 1.

11. Composés selon la revendication 10, caractérisés en ce que $R_1$ représente un phényle substitué en position 2 par un groupe —Y—halogène-alcényle en $C_2$ à $C_6$, $R_2$ représente un hydrogène, Z représente un oxygène, $R_3$ représente un hydrogène ou un alcoyle en $C_1$ à $C_4$, $R_4$ représente un alcoxy en $C_1$ à $C_4$, alcoyl-thio en $C_1$ à $C_4$, —N—di-alcoyle en $C_1$ à $C_4$, —$SO_2$—alcoyle en $C_1$ à $C_4$ ou halogène-alcoxy en $C_1$ à $C_4$ et Y représente un oxygène ou un soufre.

12. Composés selon la revendication 11, caractérisés en ce que $R_1$ représente un 2-(1,2-dichlorovinyloxy)-phényle ou 2-(1,2-dichlorovinyl-thio)-phényle.

13. Composés selon la revendication 12, caractérisés en ce que $R_1$ représente un 2-(1,2-dichlorovinyloxy)-phényle.

14. Composés selon la revendication 10, caractérisés en ce que $R_1$ représente un phényle substitué en position 2 par un fluor, chlore, nitro, alcoxycarbonyle en $C_1$ à $C_4$, alcoxy-alcoxy en $C_2$ à $C_5$, halogène-alcoxy en $C_1$ à $C_4$, halogène-alcoyl-thio en $C_1$ à $C_4$, halogène-alcoyle en $C_1$ à $C_4$ ou —Y—halogène-alcényle en $C_2$ à $C_6$, $R_2$ représente un hydrogène, Z représente un oxygène, $R_3$ représente un hydrogène ou un alcoyle en $C_1$ à $C_4$, $R_4$ représente —X—$CHF_2$ et X et Y représentent indépendamment l'un de l'autre un oxygène ou un soufre.

15. Composés selon la revendication 14, caractérisés en ce que X représente un oxygène.

16. Composés de formule I selon la revendication 1, choisis dans la série :

N-(2-méthoxycarbonylphényl-sulfonyl)-N'-(1-méthyl-5-difluorométhoxy-1,2,4-triazol-3-yl)-urée.
N-(2-(1,2-dichlorovinyloxy)-phényl-sulfonyl)-N'-(1,5-diméthyl-1,2,4-triazol-3-yl)-urée.
N-(2-(1,2-dichlorovinyloxy)-phényl-sulfonyl)-N'-(5-éthyl-1-méthyl-1,2,4-triazol-3-yl)-urée.
N-(2-(1,2-dichlorovinyloxy)-phényl-sulfonyl)-N'-(1-méthyl-5-isopropyl-1,2,4-triazol-3-yl)-urée,
N-(2-(1,2-dichlorovinyloxy)-phényl-sulfonyl)-N'-(1-méthyl-5-cyclopropyl-1,2,4-triazol-3-yl)-urée.
N-(2-(1,2-dichlorovinyloxy)-phényl-sulfonyl)-N'-(1-méthyl-5-méthyl-thio-1,2,4-triazol-3-yl)-urée.
N-(2-(1,2-dichlorovinyloxy)-phényl-sulfonyl)-N'-(5-diméthylamino-1-méthyl-1,2,4-triazol-3-yl)-urée.
N-(2-(1,2-dichlorovinyloxy)-phényl-sulfonyl)-N'-(1-éthyl-5-méthyl-1,2,4-triazol-3-yl)-urée.
N-(2-(1,2-dichlorovinyloxy)-phényl-sulfonyl)-N'-(1,5-diéthyl-1,2,4-triazol-3-yl)-urée.
N-(2-(1,2-dichlorovinyloxy)-phényl-sulfonyl)-N'-(5-méthoxy-1-méthyl-1,2,4-triazol-3-yl)-urée.
N-(2-(1,2-dichlorovinyloxy)-phényl-sulfonyl)-N'-(1-méthyl-5-difluorométhoxy-1,2,4-triazol-3-yl)-urée.
N-(2-(1,2-dichlorovinyloxy)-phényl-sulfonyl)-N'-(1,3-diméthyl-1,2,4-triazol-5-yl)-urée.

17. Procédé de préparation de composés de formule I, caractérisé en ce qu'on fait réagir un dérivé de phényl-sulfonyle de formule II

$$R_1—SO_2W \qquad (II)$$

avec un composé de formule

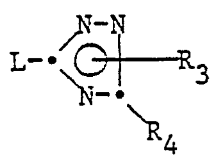

(III)

où L et W représentent l'un des groupes —$NH_2$— —N=C=Z ou —$NR_2$—CZ—OR ; les substituants $R_1$, $R_2$, $R_3$, $R_4$ et Z ont les significations données sous la formule I, R représente un radical aliphatique ou aromatique ; avec la précision que les réactifs de formules II et III sont toujours choisis de manière qu'une fonction amino réagisse avec un groupe isocyanato, isothiocyanato ou avec un groupe (—$NR_2$—CZ—OR).

18. Procédé selon la revendication 17, caractérisé en ce que R représente un alcoyle en $C_1$ à $C_4$, phényle ou benzyle.

19. Agent herbicide, bactéricide et/ou de régulation de la croissance des plantes, caractérisé en ce qu'il contient comme composant actif au moins un composé de formule I selon la revendication 1.

20. Agent selon la revendication 19, caractérisé en ce qu'il contient de 0,1 à 95 % en poids d'un composé de formule I selon la revendication 1 et de 1 à 99,9 % en poids d'additifs, dont 0,1 à 25 % en poids d'un agent tensio-actif.

21. Application d'un composé de formule I selon la revendication 1 à la lutte contre les espèces

végétales indésirables dans les cultures de plantes utiles et/ou à la régulation de la croissance végétale des plantes utiles.

22. Application selon la revendication 21 pour la lutte sélective contre les mauvaises herbes.

23. Procédé de lutte en pré- ou en post-levée contre les mauvaises herbes et/ou pour influer sur la croissance des plantes utiles, caractérisé en ce qu'on applique une quantité efficace d'une matière active de formule I selon la revendication 1 sur les plantes nuisibles ou les plantes utiles, des parties de ces plantes ou l'endroit où elles se trouvent.

24. Application d'un composé de formule IX selon la revendication 5 à la lutte contre les bactéries phytopathogènes.

25. Application selon la revendication 24, caractérisée en ce que les bactéries appartiennent à la famille des xanthomonocées ou pseudomonacées.

26. Procédé de lutte contre les bactéries phytopathogènes, caractérisé en ce qu'on applique une quantité efficace d'une matière active de formule IX sur les plantes utiles à protéger, les diverses parties de ces plantes ou l'endroit où elles se trouvent.

**Revendications** (pour l'Etat contractant AT)

1. Agent herbicide et/ou de régulation de la croissance végétale, caractérisé en ce qu'il contient comme composant actif au moins une N-arylsulfonyl-N'-triazolyl-urée de formule I

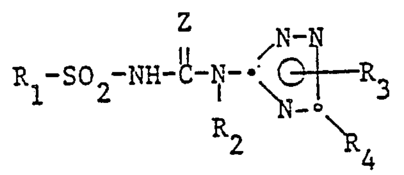

où

$R_1$ représente un radical de formule

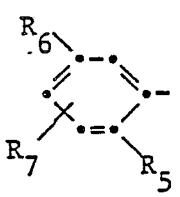

ou

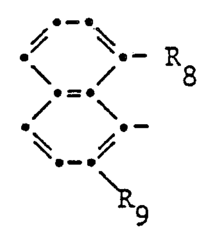

$R_2$ et $R_3$ représentent indépendamment l'un de l'autre un hydrogène ou un alcoyle en $C_1$ à $C_6$,

$R_4$ représente un hydrogène, halogène, alcoyle en $C_1$ à $C_6$, halogène-alcoyle en $C_1$ à $C_6$, cyclopropyle, —$NR_{10}R_{11}$ ou —X—$R_{12}$ et

Z représente un oxygène ou un soufre, ou

$R_5$ et $R_9$ représentent un hydrogène, halogène, cyano, nitro, alcoyle en $C_1$ à $C_6$ halogène-alcoyle en $C_1$ à $C_6$, alcényle en $C_2$ à $C_6$ éventuellement substitué par de 1 à 3 atomes d'halogène, —CO—$R_{13}$, —$NR_{10}R_{11}$, —$SO_2$—$C_1$-$C_4$-halogène-alcoxy, —$SO_2$—$NR_{10}R_{11}$, —NH—$SO_2$—$R_{15}$, N(C alcoyle en $C_1$ à $C_4$), ou un radical —Y—$R_{14}$ éventuellement substitué par un ou plusieurs substituants, de la série halogène, alcoxy en $C_1$ à $C_4$, alcoyl-thio en $C_1$ à $C_4$, alcoyl-sulfinyle en $C_1$ à $C_4$, alcoyl-sulfonyle en $C_1$ à $C_4$, halogène-alcoxy en $C_1$ à $C_4$, halogène-alcoyl-thio en $C_1$ à $C_4$, halogène-alcoyl-sulfinyle en $C_1$ à $C_4$ ou halogène-sulfonyle en $C_1$ à $C_4$,

$R_6$ représente un hydrogène, halogène, nitro ou alcoxy en $C_1$ à $C_6$,

$R_7$ et $R_8$ représentent un hydrogène, halogène, nitro, halogène-alcoyle en $C_1$ à $C_6$, —$NH_2$, alcoyle en $C_1$ à $C_6$, alcoxy en $C_1$ à $C_6$, alcoylthio en $C_1$ à $C_6$, alcoyl-sulfonyle en $C_1$ à $C_6$ ou alcoyl-sulfonyle en $C_1$ à $C_6$,

$R_{10}$ et $R_{11}$ représentent indépendamment l'un de l'autre un hydrogène, alcoyle en $C_1$ à $C_4$ ou cyclopropyle ou $R_{10}$ et $R_{11}$ représentent ensemble un pont alcoylène éventuellement substitué par un méthyle ou interrompu par un oxygène, soufre, —SO—, —$SO_2$—, —NH— ou —N(alcoyle en $C_1$ à $C_4$),

$R_{12}$ représente un alcoyle en $C_1$ à $C_4$, halogène-alcoyle en $C_1$ à $C_4$ ou alcoxy-alcoyle avec au plus 4 atomes de carbone,

$R_{13}$ représente un hydrogène, alcoyle en $C_1$ à $C_4$, alcényloxy en $C_3$ à $C_6$, alcynyloxy en $C_3$ à $C_6$, halogène-alcoyle en $C_1$ à $C_4$, alcoylthio en $C_1$ à $C_6$, phénoxy, benzyloxy, —$NR_{10}R_{11}$ ou alcoxy en $C_1$ à $C_6$ éventuellement substitué par 1 à 3 atomes d'halogène,

$R_{14}$ représente un alcoyle en $C_1$ à $C_6$, alcényle en $C_2$ à $C_6$ ou alcynyle en $C_3$ à $C_6$,

$R_{15}$ représente un alcoyle en $C_1$ à $C_6$ ou un halogène-alcoyle en $C_1$ à $C_6$ et

X et Y représentent indépendamment l'un de l'autre un oxygène, soufre, un pont sulfinyle ou sulfonyle, ainsi que les sels de ces composés ;

avec la précision que

a) $R_1$ n'a la signification indiquée que lorsque $R_4$ représente le groupe —X—$CHF_2$, et

b) $R_4$ n'a la signification indiquée que lorsque $R_1$ représente le noyau phényle substitué et $R_5$ représente le groupe —Y—halogène-alcényle en $C_2$ à $C_6$.

2. Agent selon la revendication 1, caractérisé en ce qu'il contient de 0,1 à 95 % en poids d'un composé de formule I selon la revendication 1 et de 1 à 99,9 % en poids d'additifs, dont 0,1 à 25 % en poids d'un agent tensio-actif.

3. Agent selon la revendication 1, caractérisé en ce que

$R_1$ représente un radical de formule

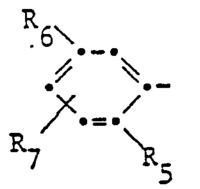

ou

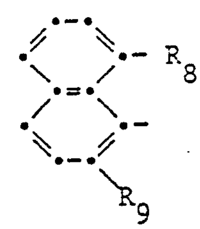

$R_2$ et $R_3$ représentent indépendamment l'un de l'autre un hydrogène ou un alcoyle en $C_1$ à $C_4$

$R_4$ représente un hydrogène, halogène, alcoyle en $C_1$ à $C_4$, halogène-alcoyle en $C_1$ à $C_4$, cyclopropyle, —$NR_{10}R_{11}$ ou —X—$R_{12}$ et

Z représente un oxygène ou un soufre,

où

$R_5$ représente un hydrogène, halogène, cyano, nitro, alcoyle en $C_1$ à $C_4$, halogène-alcoyle en $C_1$ à $C_4$, alcényle en $C_2$ à $C_6$, —CO—$R_{13}$, —$NR_{10}R_{11}$ ou un radical —Y—$R_{14}$ éventuellement substitué par un ou plusieurs substituants de la série halogène, alcoxy en $C_1$ à $C_4$, alcoylthio en $C_1$ à $C_4$, alcoyl-sulfinyle en $C_1$ à $C_4$, alcoyl-sulfonyle en $C_1$ à $C_4$, halogène-alcoxy en $C_1$ à $C_4$, halogène-alcoyl-thio en $C_1$ à $C_4$, halogène-alcoyl-sulfinyle en $C_1$ à $C_4$ ou halogène-sulfonyle en $C_1$ à $C_4$,

$R_6$ représente un hydrogène, halogène, nitro ou alcoxy en $C_1$ à $C_4$,

$R_7$ représente un hydrogène, halogène, nitro, halogène-alcoyle en $C_1$ à $C_4$, —$NH_2$, alcoyle en $C_1$ à $C_4$, alcoxy en $C_1$ à $C_4$, alcoyl-thio en $C_1$ à $C_3$, alcoyl-sufinyle en $C_1$ à $C_3$ ou alcoyle sulfonyle en $C_1$ à $C_3$,

$R_{10}$ et $R_{11}$ représentent indépendamment l'un de l'autre un hydrogène, alcoyle en $C_1$ à $C_3$, cyclopropyle, alcényle en $C_2$ à $C_6$ ou alcynyle en $C_3$ à $C_6$ ou $R_{10}$ et $R_{11}$ représentent ensemble un pont alcoylène en $C_4$ à $C_6$ éventuellement substitué par un méthyle ou interrompu par un méthylène, soufre, —SO—, —$SO_2$—, —NH— ou —N(alcoyle en $C_1$ à $C_4$),

$R_{12}$ représente un alcoyle en $C_1$ à $C_4$, halogène-alcoyle en $C_1$ à $C_4$ ou alcoxy-alcoyle avec au plus 4 atomes de carbone,

$R_{13}$ représente un hydrogène, alcoyle en $C_1$ à $C_4$, alcényloxy en $C_3$ à $C_5$, alcynyloxy en $C_3$ à $C_5$, alcoyl-thio en $C_1$ à $C_5$, phénoxy, benzyloxy, —$NR_{10}R_{11}$ ou alcoxy en $C_1$ à $C_5$ éventuellement substitué par de 1 à 3 atomes d'halogène,

$R_{14}$ représente un alcoyle en $C_1$ à $C_6$, alcényle en $C_2$ à $C_6$ ou alcynyle en $C_3$ à $C_6$ et

X et Y représentent indépendamment l'un de l'autre un oxygène, soufre, un pont sulfinyle ou un pont sulfonyle, où $R_8$ a la même signification que $R_7$ et $R_9$ a la même signification que $R_5$ ;

ainsi que les sels de ces composés, avec cette précision que

a) $R_1$ n'a la signification indiquée que lorsque $R_4$ représente le groupe —X—$CHF_2$, et que

b) $R_4$ n'a la signification indiquée que lorsque $R_1$ représente le noyau phényle substitué et $R_5$ représente le groupe —Y—halogène-alcényle en $C_2$ à $C_6$.

4. Agent selon la revendication 1, caractérisé en ce que le substituant $R_1$ représente le groupe

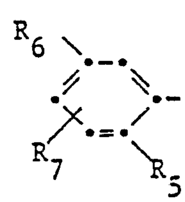

où $R_5$ représente le groupe —Y-halogène-alcoyle en $C_2$ à $C_6$ et les substituants ont les significations données pour la formule I.

5. Agent selon la revendication 1, caractérisé en ce que $R_4$ représente le groupe —X—$CHF_2$.

6. Agent selon la revendication 4, caractérisé en ce qu'ils correspondent à la formule IX

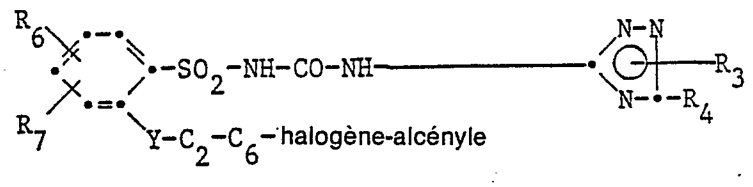

où $R_3$ représente un hydrogène ou un alcoyle en $C_1$ à $C_4$, $R_4$ représente un alcoxy en $C_1$ à $C_4$, alcoylthio en $C_1$ à $C_4$, —N—di-alcoyle en $C_1$ à $C_4$, —$SO_2$-alcoyle en $C_1$ à $C_4$ ou halogène-alcoxy en $C_1$ à $C_4$ et Y représente un oxygène ou un soufre et $R_6$ et $R_7$ ont la signification donnée sous la formule I dans la revendication 1.

7. Agent selon la revendication 6, caractérisé en ce que $R_6$ et $R_7$ représentent un hydrogène.

8. Agent selon la revendication 7, caractérisé en ce que le groupe —Y—halogène-alcényle en $C_2$ à $C_6$ représente un 1,2-dichlorovinyloxy ou un 1,2-dichlorovinylthio.

9. Agent selon la revendication 8, caractérisé en ce que le groupe —Y—halogène-alcényle en $C_2$ à $C_6$ représente un 1,2-dichlorovinyloxy.

10. Agent selon la revendication 1, caractérisé en ce que $R_1$ représente un phényle substitué en position 2 par un fluor, chlore, nitro, alcoxycarbonyle en $C_1$ à $C_4$, alcoxy-alcoxy en $C_2$ à $C_5$, halogène-alcoxy en $C_2$ à $C_5$, halogène-alcoxy en $C_1$ à $C_4$, halogène-alcoyl-thio en $C_1$ à $C_4$, halogène-alcoxy en $C_1$ à $C_4$ ou —Y—halogène-alcényle en $C_2$ à $C_6$, $R_2$ représente un hydrogène, Z représente un oxygène $R_3$ représente un hydrogène ou un alcoyle en $C_1$ à $C_4$, $R_4$ représente —X—$CHF_2$ et X et Y représentent indépendamment l'un de l'autre un oxygène ou un soufre.

11. Agents selon la revendication 1, caractérisé en ce qu'ils correspondent à la formule Ia

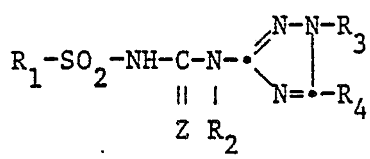

où $R_1$ à $R_4$ et Z ont la signification donnée sous la formule I dans la revendication 1.

12. Agent selon la revendication 11, caractérisé en ce que $R_1$ représente un phényle substitué en position 2 par un groupe —Y—halogène-alcényle en $C_2$ à $C_6$, $R_2$ représente un hydrogène, Z représente un oxygène, $R_3$ représente un hydrogène ou un alcoyle en $C_1$ à $C_4$, $R_4$ représente un alcoxy en $C_1$ à $C_4$, alcoyl-thio en $C_1$ à $C_4$, —N—di-alcoyle en $C_1$ à $C_4$, —$SO_2$-alcoyle en $C_1$ à $C_4$ ou halogène-alcoxy en $C_1$ à $C_4$ et Y représente un oxygène ou un soufre.

13. Agent selon la revendication 12, caractérisé en ce que $R_1$ représente un 2-(1,2-dichlorovinyloxy)-phényle ou 2-(1,2-dichlorovinyl-thio)-phényle.

14. Agent selon la revendication 13, caractérisé en ce que $R_1$ représente un 2-(1,2-dichlorovinyloxy)-phényle.

15. Agent selon la revendication 11, caractérisé en ce que $R_1$ représente un phényle substitué en position 2 par un fluor, chlore, nitro, alcoxycarbonyle en $C_1$ à $C_4$, alcoxy-alcoxy en $C_2$ à $C_5$, halogène-alcoxy en $C_1$ à $C_4$, halogène-alcoyl-thio en $C_1$ à $C_4$, halogène-alcoyle en $C_1$ à $C_4$, halogène-alcoyl-thio en $C_1$ à $C_4$, halogène-alcoyle en $C_1$ à $C_4$ ou —Y—halogène-alcényle en $C_2$ à $C_6$, $R_2$ représente un hydrogène, Z représente un oxygène, $R_3$ représente un hydrogène ou un alcoyle en $C_1$ à $C_4$, $R_4$ représente —X—$CHF_2$ et X et Y représentent indépendamment l'un de l'autre un oxygène ou un soufre.

16. Agent selon la revendication 15, caractérisé en ce que X représente un oxygène.

17. Agent selon la revendication 1, caractérisé en ce que les matières actives sont choisies dans la série

N-(2-méthoxycarbonylphényl-sulfonyl)-N'-(1-méthyl-5-difluorométhoxy-1,2,4-triazol-3-yl)-urée.
N-[2-(1,2-dichlorovinyloxy)-phényl-sulfonyl]-N'-(1,5-diméthyl-1,2,4-triazol-3-yl)-urée.
N-[2(-1,2-dichlorovinyloxy)-phényl-sulfonyl]-N'-(5-éthyl-1-méthyl-1,2,4-triazol-3-yl)-urée.
N-[2-(1,2-dichlorovinyloxy)-phényl-sulfonyl]-N'-(1-méthyl-5-isopropyl-1,2,4-triazol-3-yl)-urée.
N-[2-(1,2-dichlorovinyloxy)-phényl-sulfonyl]-N'-(1-méthyl-5-cyclopropyl-1,2,4-triazol-3-yl)-urée.
N-[2-(1,2-dichlorovinyloxy)-phényl-sulfonyl]-N'-(1-méthyl-5-méthyl-thio-1,2,4-triazol-3-yl)-urée.
N-[2-(1,2-dichlorovinyloxy)-phényl-sulfonyl]-N'-(5-diméthylamino-1-méthyl-1,2,4-triazol-3-yl)-urée.
N-[2-(1,2-dichlorovinyloxy)-phényl-sulfonyl]-N'-(1-éthyl-5-méthyl-1,2,4-triazol-3-yl)-urée.
N-[2-(1,2-dichlorovinyloxy)-phényl-sulfonyl]-N'-(1,5-diéthyl-1,2,4-triazol-3-yl)-urée.
N-[2-(1,2-dichlorovinyloxy)-phényl-sulfonyl]-N'-(5-méthoxy-1-méthyl-1,2,4-triazol-3-yl)-urée.

N-[2-(1,2-dichlorovinyloxy)-phényl-sulfonyl]-N'-(1-méthyl-5-difluorométhoxy-1,2,4-triazol-3-yl)-urée.
N-[2-(1,2-dichlorovinyloxy)-phényl-sulfonyl]-N'-(1,3-diméthyl-1,2,4-triazol-5-yl)-urée.

18. Procédé de préparation de composés de formule I, caractérisé en ce qu'on fait réagir un dérivé de phényl-sulfonyle de formule II

$$R_1—SO_2W \qquad (II)$$

avec un composé de formule

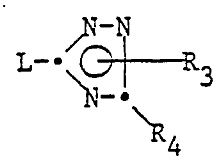

(III)

où L et W représentent l'un des groupes —$NH_2$— —N=C=Z ou —$NR_2$—CZ—OR ; les substituants $R_1$, $R_2$, $R_3$, $R_4$ et Z ont les significations données sous la formule I, R représente un radical aliphatique ou aromatique ; avec la précision que les réactifs de formules II et III sont toujours choisis manière qu'une fonction amino réagisse avec un groupe isocyanato, isothiocyanato ou avec un groupe —$NR_2$—CZ—OR.

19. Procédé selon la revendication 18, caractérisé en ce que R représente un alcoyle en $C_1$ à $C_4$, un phényle ou un benzyle.

20. Application d'un composé de formule I selon la revendication 1 pour lutter contre les espèces végétales indésirables dans les cultures de plantes utiles et/ou pour la régulation de la croissance végétale des plantes utiles.

21. Application selon la revendication 20 pour la lutte sélective contre les mauvaises herbes.

22. Procédé de lutte en pré- ou en post-levée contre les mauvaises herbes et/ou pour agir sur la croissance des plantes utiles, caractérisé en ce qu'on applique une quantité efficace d'une matière active de formule I selon la revendication 1, sur les plantes nocives ou les plantes utiles, leurs parties de plantes ou l'endroit où elles se trouvent.

23. Application d'un composé de formule IX selon la revendication 6 pour combattre les bactéries phytopathogènes.

24. Application selon la revendication 23, caractérisée en ce que les bactéries appartiennent à la famille des xanthomonocées ou pseudomonacées.

25. Procédé de lutte contre les bactéries phytopathogènes, caractérisé en ce qu'on applique une quantité efficace d'une matière active de formule IX sur les plantes utiles à protéger, les diverses parties de ces plantes ou l'endroit où elles se trouvent.